(19)

**Europäisches Patentamt**
**European Patent Office**
**Office européen des brevets**

(11) **EP 4 202 440 A1**

(12) **EUROPEAN PATENT APPLICATION**

(43) Date of publication:
**28.06.2023 Bulletin 2023/26**

(21) Application number: **21216747.2**

(22) Date of filing: **22.12.2021**

(51) International Patent Classification (IPC):
**G01N 33/574** (2006.01)

(52) Cooperative Patent Classification (CPC):
**G01N 33/57426;** G01N 2800/52; G01N 2800/60

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Max-Planck-Gesellschaft zur Förderung der Wissenschaften e.V.**
**80539 München (DE)**
• **Johann-Wolfgang-Goethe-Universität Frankfurt am Main**
**60323 Frankfurt am Main (DE)**
• **Deutsches Krebsforschungszentrum Heidelberg Stiftung des öffentlichen Rechts (DKFZ)**
**69120 Heidelberg (DE)**

(72) Inventors:
• **Mann, Matthias**
**82152 Planegg/Martinsried (DE)**
• **Jayavelu, Ashok Kumar**
**81375 München (DE)**
• **Urlaub, Henning**
**37083 Göttingen (DE)**
• **Oellerich, Thomas**
**60590 Frankfurt am Main (DE)**
• **Wolf, Sebastian**
**60596 Frankfurt am Main (DE)**
• **Serve, Hubert**
**60431 Frankfurt am Main (DE)**
• **Büttner, Florian**
**60316 Frankfurt am Main (DE)**

(74) Representative: **Dentons Patent Solutions Rechtsanwaltsgesellschaft mbH Jungfernturmstraße 2 80333 München (DE)**

(54) **PROTEOMICS-BASED STRATIFICATION OF ACUTE MYELOID LEUKAEMIA**

(57) The present invention relates to a method of identifying sub-forms of acute myeloid leukemia (AML), said method comprising (a) determining protein expression levels of a plurality of proteins in a cohort of patients suffering from AML; and (b) classifying said patients according to said protein expression levels; thereby obtaining classes of patients which define sub-forms of AML.

Figure 2

**EP 4 202 440 A1**

**Description**

The present invention relates to a method of identifying sub-forms of acute myeloid leukaemia (AML), said method comprising (a) determining protein expression levels of a plurality of proteins in a cohort of patients suffering from AML; and (b) classifying said patients according to said protein expression levels; thereby obtaining classes of patients which define sub-forms of AML.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Acute myeloid leukemia (AML) is a clinically and genetically heterogeneous disease characterized by bone-marrow infiltration with immature leukemic blasts leading to bone-marrow failure. Genomic studies have identified driver events in AML mutations and cytogenetic aberrations, which define prognostic subgroups and suggest drug targets (Döhner et al., 2017). Knowledge of the individual genomic features of a given AML has become valuable for decisions regarding therapy stratification (DiNardo et al., 2018; Stone et al., 2017). However, even though genomics has helped to resolve some of the clinical heterogeneity of AML and has revealed specific drug targets, there remain many challenges. First, there continues to be a mismatch between the genomic risk stratification and clinical outcome for some individual patients. For example, a considerable number of patients with genetic features associated with a favorable outcome (e.g. NPM1-mutant and CBF leukemias) in fact show poor outcome under standard therapy, highlighting our incomplete understanding of the pathophysiology of AML and its response to therapy (Lachowiez et al., 2020; Solh et al., 2014). Secondly, most mutated effectors in AML are still not druggable, and their impact on downstream processes, such as protein networks and particularly drug response, remains poorly understood (Shafer and Grant, 2016). Hence, there is a strong medical need to elucidate the mechanisms governing the maintenance of AML as a basis for identifying new drug targets. Moreover, future efforts are needed to refine AML classification beyond genomic features, to improve biomarker identification and therapy stratification.

More recently, the combination treatment with azacytidine plus the BCL2 antagonist venetoclax showed strong efficacy in AML patients who are ineligible for intensive induction therapy (DiNardo et al., 2020). This efficacious "mutation-agnostic" approach is now regarded as the preferred therapy for such patients, and has become an important backbone for the development of future therapies. However, despite the high efficacy of this combination treatment, biomarkers for response prediction are currently lacking, especially because genomic markers showed only limited predictive value (Bhatt et al., 2020). More accurate response predictions, which go beyond genomic aberrations, are needed.

Proteomics has long been seen as a promising technique complementary to genomics for elucidating cancer biology and identifying diagnostic and predictive biomarkers (Chopra et al., 2013). However, there are still only very few if any clinically relevant biomarkers and disease subtypes that have been identified by this technology. In recent years the constant improvement of proteomic techniques has paved the way for very deep quantitative profiling of cellular proteomes now making it possible to elucidate the molecular pathophysiology of cancer beyond mere genomics, and to systematically define proteogenomic disease subtypes and biomarkers with clinical relevance (Coscia et al., 2018).

In view of the prior art and its shortcomings, the technical problem underlying the present invention can be seen as the provision of improved means and methods for identifying sub-forms of AML, diagnosing AML and treating AML as well as of sub-forms thereof.

These technical problems have been solved by the subject-matter of the claims, as demonstrated by the Examples.

Accordingly, in a first aspect, the invention provides a method of identifying sub-forms of acute myeloid leukaemia (AML), said method comprising (a) determining protein expression levels of a plurality of proteins in a cohort of patients suffering from AML; and (b) classifying said patients according to said protein expression levels; thereby obtaining classes of patients which define sub-forms of AML.

Acute myeloid leukaemia (AML) is a form of leukaemia or blood cancer. It is characterized by hyper-proliferation of the myeloid line of blood cells. Abnormal cells build up in the bone marrow and the blood. Owing to its acute nature, AML, if untreated, is generally associated with a short life expectancy in the range of months or even weeks. Whether a patient suffers from AML may be determined by in vitro analysis of a sample, preferably a blood sample or a bone marrow sample taken from said patient. Generally, an AML patient will also present typical clinical symptoms such as tiredness, shortness of breath, and easy bruising and bleeding.

A stratification of AML patients may be based on various parameters. A distinction between AML patients based on any parameter defines sub-forms of AML. Of particular interest are sub-forms which can be distinguished with regard course of the disease, prognosis, life expectancy, clinical symptoms, and treatment of choice. Preferred sub-forms in accordance with this invention are given below. Of note, while sub-forms of AML in accordance with the invention are of clinical relevance, they can only be determined by analysis of protein expression data.

Knowledge of sub-forms is useful for the attending doctor or clinician, and may help to make better decisions, e.g. in terms of diagnosis, monitoring and treatment. Experience tells that even patients such as AML patients which may appear uniform from a clinical standpoint response differently to a given treatment. What might be a treatment of first choice for one sub-group of patients (which is suffering from one particular sub-form of AML) may have no effect in another sub-group or even deteriorate the condition of the affected patients, be it because such treatment fails to trigger any beneficial effect such that only side-effects become apparent, or even because the treatment deteriorates the condition it intends to ameliorate. This applies also to the proteomics-based stratification of AML patients as defined in the first aspect of the invention.

The method employed for classifying is not particularly limited. It may be, and preferably is computer-implemented. Preferably, classifying is unsupervised. A preferred method of classifying is clustering. Preferred is unsupervised clustering. Computational software such as Perseus, R Studio, and Python. Statistical approach is Cox regression model may be used. Preferred is Perseus (see Tyanova et al., Nature Methods 2016, 13, 731-740; Cox and Mann, BMC Bioinformatics 2012, 13 Suppl 16: S12; and http://maxquant.net).

Further data integration methods for disease subtyping, classification, and biomarker identification can be of following categories: network, Bayesian, fusion, similarity-based, correlation-based, and other multivariate methods (PMID: 32076369). For multi-omics data integration approaches most preferred methods are similarity based, dimension reduction based and statistical modelling-based such as MOFA (multi-omics factor analysis). Preferred is MOFA (PMID: 29925568).

Patient data represent data points in a multidimensional space, the number of dimensions of said space generally being the number of parameters for which measurements or information is available. Data for all parameters may be available for all patients considered, but do not have to be. Classifying may also be applied to incomplete or sparse data sets. Representation in a multidimensional space allows to determine distances between patients. Various distance metrics have been described, including Euclidean distance, squared Euclidian distance, maximum distance, Mahalanobis distance, Manhattan distance and Pearson distance. Preferred is Euclidian distance. When classifying or clustering yields distinguishable classes or clusters, distances between patients within a given class or cluster are generally smaller than distances between patients belonging to distinct classes or clusters.

In terms of input data, said data may be confined to protein expression data, but may also include data from other domains such as mRNA expression data, cytogenetic profiles (such as aberrant chromosomal structures), information about mutations and/or results of microscopic analysis of cells.

A cohort of patients is a plurality of patients. As generally in statistics, the larger such cohort is, the more reliable are the conclusions derived from analysing the data characterizing the patients of said cohort. Preferred cohort sizes are at least 20, at least 50, at least 100, at least 200 or at least 500. Exemplary cohort sizes are given in the Examples. Preferably, patients of a cohort are selected in an unbiased or random manner. This is a means of ensuring, as good as possible in view of the size of a cohort, that patients are comprised in said cohort which differ from each other at the molecular, cellular and/or clinical level, and/or that said cohort is a good representation of the entirety of AML patients at a given point in time.

Protein expression may be determined by any suitable methods. Preferred methods or assays are disclosed further below. Particularly preferred is mass spectrometry (MS). Mass spectrometry has evolved over the years to become the method of choice for high throughput and high precision proteomics.

The present inventors surprisingly discovered that in the domain of AML unexpectedly large differences can be observed between gene expression levels determined at the mRNA level (which reflect transcription regulation) and at the protein level (which reflect translation regulation and post-translational events such as protein lifetime). This is even more surprising given that AML and stratification of AML patients have been the subject of intense research over decades (see the publications cited in the introductory part above). Of note, already a multitude of parameters has been relied upon when defining AML sub-groups in the past, said parameters including mRNA expression, and furthermore cytogenetics and analysis of disease-associated mutations.

Using unsupervised clustering of proteomic data (see Examples), the present inventors were able to identify a sub-form of AML which has not been recognized previously. Of note, said new sub-form is of immediate clinical relevance.

While the method of the first aspect relates to a method of identifying sub-forms of AML based on proteomic data, the present invention, based on findings obtained when applying the method of the first aspect, also permits that a prediction and a therapy recommendation for new patient is provided. Such predictions and recommendations, which are subject of further aspects of this invention disclosed further below, provide for tailored therapy of such a patient on the basis of protein expression measurements.

In a preferred embodiment, said plurality of proteins comprises or consists of proteins detectable by proteomics of AML cells including a single AML cell, preferably mitochondrial proteins detectable by said proteomics. AML cells are of myeloid origin.

Single cell proteomics currently provides for about 300 to 1000 such as 400 to 500 proteins being detectable when using material from one single cell for proteomics, proteomics preferably being implemented by mass spectrometry.

Among those, about 5% to 15% such as about 10% are generally mitochondrial proteins.

The inventors found that for defining sub-forms, including new sub-forms of AML, a subset of proteins, viz. mitochondrial proteins is sufficient. Owing to the dominant contribution of mitochondrial proteins to the definition of said sub-form, corresponding patients are also referred to herein as belonging to a "mitochondrial cluster" or the "mito cluster". The term "mitochondrial" as used herein refers to the subcellular localization in the mitochondria. As such, the terms embraces both proteins encoded in the nucleus as well as in the mitochondrial genome, provided that the encoded protein is located or predominantly located in the mitochondria. Mitochondrial proteins in accordance with the above definition are known, see, for example, https://www.broadinstitute.org/files/shared/metabolism/mitocarta/human.mitocarta3.0.html

Preferably, protein expression levels of at least 3, at least 4, at least 5, at least 6, at least 7, at least 8, at least 9, at least 10, at least 15, at least 20, at least 30, at least 40, at least 50, at least 100, at least 200, at least 300, at least 400, or at least 500 mitochondrial proteins are determined. Protein expression may also be determined for all or substantially all mitochondrial proteins; see the above website for a list of mitochondrial proteins. There is no requirement in that respect, though, because also use of small sets of mitochondrial proteins as disclosed above is sufficient. This applies to aspects of the invention where protein expression levels of mitochondrial proteins are to be determined or considered.

In a preferred embodiment, said sub-forms differ from each other with regard to at least one of overall survival, prognosis, response to chemotherapy, and treatment of choice. In other words, while protein expression data are required to define the sub-form which is the mito cluster, such sub-form is also clinically distinct from other sub-forms. Of note, while patient-to-patient variations of life expectancy are well established for AML, an inference of the mito cluster from survival data is not possible, and, even if it were possible would be of limited value since any information gained from retrospective survival analysis would not provide benefit to the analyzed patients. The mito cluster of patients may be viewed as a subset of patients with low life expectancy, said subset being characterized by a distinct molecular signature. Knowing that a patient has said signature is of immediate benefit because such patient qualifies, as disclosed further below, for a specific therapy.

In a second aspect, the present invention provides a use of a set of proteins for determining prognosis of and/or treatment of choice for a patient suffering from AML or for selecting said patient for a specific therapy, wherein said set of proteins comprises or consists of (i) at least three mitochondrial proteins; or (ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

This aspect mirrors the relevance of specific protein sets in accordance with the invention. In terms of practical applications, providing information about these protein sets enables the skilled person to implement diagnostic and therapeutic applications which are subject of further aspects disclosed below. Implementation of such application may require previous calibration of an assay which calibration is subject of a further aspect of the invention; see further below.

In addition to the finding of the relevance of mitochondrial proteins, it has been found that it is a subset of 27 proteins in accordance with item (ii) as defined above or any subset of at least 3, such as 3, 4, 5, 6, 7, 8, 9,10,11, 12, 13, 14 or 15 of said 27 proteins which captures the relevant variance in the above mentioned data space and allows to determine prognosis or treatment of choice, and, related thereto, assign a patient to said mito cluster. Any such subset is a subset in accordance with the present invention. Of note, said subset of 27 proteins does not exclusively consist of mitochondrial proteins.

The names of the proteins given in item (ii) are common in the art. Further information about these proteins can be found in curated protein databases such as Uniprot, Expasy, and phosphositeplus.

Particularly preferred subsets of said 27 proteins are the subject of preferred embodiments disclosed further below.

"Treatment of choice" designated a treatment with superior outcome as compared to other treatment options for a patient with a given proteomics profile. While classical chemotherapy including induction chemotherapy may still be appropriate for certain sub-forms of AML, this does not apply the mito cluster of patients. For these patients, treatment with agents disclosed further below provides for an outcome which is superior to the outcome achievable with chemotherapy.

In a third aspect, the present invention provides a method of calibrating a method of determining prognosis and/or treatment of choice of a patient suffering from AML, said method comprising

(a) determining the protein expression levels of a set of proteins in a cohort of patients suffering from AML;
(b) comparing expression levels of individual patients to average expression levels of said set of proteins in said cohort;
(c) correlating expression levels which deviate from said average expression levels with observed overall survival and/or response to chemotherapy;

wherein said set of proteins comprises or consists of

(i) at least three mitochondrial proteins; or
(ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, , CSK, MRPL19, NUP88 and nucleoprotein TPR.

This method details how a method of determining prognosis and/or treatment of choice may be calibrated. Similar to the use of the second aspect, the key information are the sets of proteins in accordance with the invention.

Once protein expression levels of a cohort are determined, these may be stored in a database for future use, e.g. by methods in accordance with further aspects of the present invention. Over time, such database may grow as data for further patients are being added.

In a fourth aspect, the present invention relates to a method of determining prognosis and/or response to chemotherapy of a patient suffering from AML, said method comprising

(a) determining the protein expression levels of a set of proteins in said patient;
(b) comparing said expression levels to the average expression levels of said set of proteins in a cohort of AML patients, wherein said average expression levels may be retrieved in a database;
(c) assigning bad prognosis and/or poor response to chemotherapy to said patient if said expression levels deviate from said average expression levels,

wherein said set of proteins comprises or consists of

(i) at least three mitochondrial proteins, wherein said mitochondrial proteins are predominantly elevated as compared to said average expression levels; or
(ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels.

The term "predominantly" refers to at least 50% of mitochondrial proteins the protein expression of which has been determined. More preferably are at least 60%, 2/3, or at least 70%.

Expression levels of the recited cohort may be determined when practising the method of the fourth aspect. Alternatively, they may be retrieved from a database, for example a database which has been built up when performing the method of calibrating of the third aspect. Moreover, values stored in a database may be combined with newly acquired values, be it of a single patient or of a new/further cohort.

Patients with a predicted poor response to chemotherapy are preferably subjected to an alternative treatment in accordance with the invention; see further below.

As noted above, the inventors found that mitochondrial protein expression is associated with overall survival outcome of AML patients. In particular, elevated protein expression of mitochondrial proteins is associated with bad prognosis. "Bad prognosis" refers to an expected more rapid worsening of symptoms and/or an expected shorter life expectancy as compared to average course of the disease (development or worsening of symptoms, life expectancy) in said cohort. As disclosed further below, a patient which has been determined to have worse prognosis in accordance with the method of the fourth aspect, i.e. exhibits the protein expression profile as recited in the method of the fourth aspect, is characterized in that classical chemotherapy is not the therapy of first choice. Accordingly, "bad prognosis" is associated with such patient being refractory to chemotherapy and/or said patient developing earlier relapses as compared to said cohort when treated by chemotherapy. In other words, such patients have a shorter remission time. They are also referred to as "poor responders" herein.

Throughout the disclosure, "more rapid", "earlier" and "shorter" refer to statistically significant differences. "Statistically significant" preferably means a p-value, i.e., the likelihood of observing a given difference by chance, of 0.05 or less, 0.01 or less, or $10^{-3}$ or less. The protein subset of items (i) and (ii) allows for predictive statements regarding prognosis. This applies to the entire disclosure. Similarly, "elevated", "decreased" and "deviate" in relation to expression levels refers to statistically significant higher levels as compared to cohort average values.

The term "average" refers to the central tendency of the underlying distribution of expression values across said cohort. It includes median, arithmetic mean and logarithmic mean. Preferred is arithmetic mean.

Preferred subsets of the recited 27 proteins are those defined further above as well as below. The term "cohort" has been defined above. This applies also for the remainder of the disclosure if not indicated to the contrary.

Of note, performing the method of the fourth aspect does not require that said cohort of patients is actually analysed. Instead, said average expression levels may be previously determined levels which are stored, e.g. in a database. As such, and analogous to the first aspect, the method of the second aspect is preferably computer-implemented. A computer chosen for implementation is preferably configured to have access to such database. Further aspects of computer implementation of the methods of the invention are disclosed further below.

In a fifth aspect, the present invention provides a method of selecting an AML patient for a specific therapy, said method comprising

(a) determining the protein expression levels of a set of proteins in said patient;
(b) comparing said expression levels to the average expression levels of said set of proteins in a cohort of AML patients, wherein said average expression levels may be retrieved in a database;
(c) selecting said patient for said specific therapy if said expression levels deviate from said average expression levels;

wherein said set of proteins comprises or consists of

(i) at least three mitochondrial proteins, wherein said mitochondrial proteins are predominantly elevated as compared to said average expression levels; or
(ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels; and wherein said specific therapy comprises or consists of the administration to said patient of an inhibitor of mitochondrial function, preferably of the electron transport chain (ETC), more preferably of ETC complex I.

The sub-form of AML associated with elevated protein expression of mitochondrial proteins is a case in point where standard chemotherapy, possibly the therapy of choice prior to recognition of the mito cluster by the present inventors, is indeed not the best choice. To the contrary, induction chemotherapy fails to deliver a significant improvement of patients of the mito cluster. On the other hand, venetoclax, an agent known as an inhibitor of Bcl-2, does so. More generally, the inventors found that inhibitors of mitochondrial function, preferably of the electron transport chain (ETC), more preferably of ETC complex I should be the treatment of first choice for patients with said elevated expression levels. More specifically, criteria for selecting a given patient for such therapy are analogous those set forth in the fourth aspect, and definitions given there apply mutatis mutandis.

In other words, owing to the molecular understanding of the particular AML sub-form which is associated with mitochondrial protein over-expression, unspecific chemotherapy with its known associated severe side effects can be avoided, at least for patients belonging to the mito cluster.

The method of the fifth aspect is preferably computer-implemented. For aspects of the invention relating to computer implementation, see further below.

In a preferred embodiment, said specific therapy avoids chemotherapy. The first line chemotherapeutic treatment is also referred to as induction chemotherapy. The specific therapy in accordance with the invention preferably also avoids induction chemotherapy.

Agents normally used for induction chemotherapy are cytostatic agents and inhibitors of cell division or proliferation, more specifically anthracyclins such as Donorubicin and Doxorubicin, and nucleotide analogs such as Cytarabine.

Preferably, said inhibitor to be used for said specific therapy is selected from Venetoclax, Tigecycline, Mubritinib, Rotenone, Metformin, Deguelin, IACS-010759, Oligomycin, Gboxin, Gamitrinib, TRO40303, cyclosporin A, Paclitaxel, mitocans, PBR-agonists including diazepam analogs, 3-bromopyruvate, Anthracyclines, arsenic trioxide, and 4-Quinolones such as ciprofloxacin.

Below it is indicated which aspect of mitochondrial function these agents target.

Tigecycline: mitochondrial function
Venetoclax: mitochondrial function and ETC
Mubritinib: mitochondrial function and ETC
Rotenone, Metformin, Deguelin, IACS-010759: mitochondrial function and ETC complex I
Oligomycin, Gboxin: ETC complex V

Gamitrinib: inhibitor of oxidative phosphorylation

TRO40303: binds to mitochondrial outer membrane translocator protein

cyclosporin A (CsA): inhibitor of the mitochondrial permeability transition pore (MPTP) Paclitaxel, mitocans, PBR-agonists (such as diazepam analogs), 3-bromopyruvate, anthracyclines, arsenic trioxide: induction of mitochondrial permeability transition pore complex formation and of oxidative stress

4-Quinolones (such as ciprofloxacin): mitochondrial DNA damaging agents

In a preferred embodiment of any of the above disclosed aspects of the invention, said method furthermore comprises at least one of RNA expression profiling; mutational analysis; and cytogenetic analysis.

While not being indispensable for discovery of the mito cluster (indeed any discovery of the mito cluster was not possible prior to protein expression analysis) and any applications based thereon which are subject of this invention, such further data may nevertheless be employed. The combined analysis across different domains such transcriptomics, proteomics, karyotyping, mutational analysis etc. is also referred to as multi-omics data integration approach in accordance with the invention.

In a further preferred embodiment of any of the above disclosed aspects, said method is an in vitro method and/or said determining is performed with (a) sample(s) obtained from said cohort and/or said patient. In other words, said methods may be implemented without requiring presence of a human or patient.

In a sixth aspect, the present invention provides an agent for use in a method of treating a sub-form of AML, wherein said sub-form of AML is characterized by deviant expression of a set of proteins as compared to the average expression of said set of proteins in a cohort of AML patients, wherein said set of proteins comprises or consists of

(i) at least three mitochondrial proteins, wherein said mitochondrial proteins are predominantly elevated as compared to said average expression levels; or

(ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels; and wherein said agent is an inhibitor of mitochondrial function, preferably of the electron transport chain (ETC), more preferably of ETC complex I.

While the method of the fifth aspect is directed to selecting a patient for proper therapy, the agent for use of the sixth aspect relates to the new medical use of inhibitors of mitochondrial function for the treatment of a sub-form of AML, wherein said sub-form of AML is defined in terms of a protein expression profile.

Preferred subsets of proteins are those disclosed further above. The same applies to preferred inhibitors.

Related to the sixth aspect, the present invention provides in a further aspect a method of treating a patient suffering from a sub-form of AML, said method comprising administering a therapeutically effective amount of an inhibitor of mitochondrial function, preferably of the electron transport chain (ETC), more preferably of ETC complex I, to said patient, wherein said sub-form of AML is characterized by deviant expression of a set of proteins as compared to the average expression of said set of proteins in a cohort of AML patients, wherein said set of proteins comprises or consists of at least three mitochondrial proteins, wherein said mitochondrial proteins are predominantly elevated as compared to said average expression levels; or at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels.

Analogous to said method of selecting a patient of the fifth aspect, the method of treating in accordance with the sixth aspect preferably does not include induction chemotherapy or chemotherapy.

For administration, said inhibitors may be formulated by adding pharmaceutically acceptable excipients, diluents and/or stabilizers. Administration may be oral or parenteral, intravenous or subcutaneous. Appropriate dosages can be determined by the attending physician or clinician. Dosages may, but do not have to depend on parameters such as age, sex or pre-existing conditions. Suitable doses for treatment or as starting point for optimizing a dosage regimen are between about 100 mg and about 1000 mg per day such as between about 200 mg and about 500 mg per day including about 400 mg per day. At the beginning of the treatment, such as on the first two days, lower dosages may be employed.

In a seventh aspect, the present invention provides a method of identifying a candidate therapeutic target for AML, said method comprising (a) determining the expression level of at least one protein in a sample obtained from an AML patient; (b) comparing said expression level to the average expression level of said at least one protein in a cohort of AML patients; and (c) selecting a protein with a deviant, preferably an elevated expression level as determined in step (b) as candidate therapeutic target.

This aspect is another facet arising from the present inventor's surprising discovery that in the field of AML transcription levels and protein levels may differ widely. As a consequence, the proteomics approach employed by the present inventors may open avenues to the discovery of potential therapeutic targets which previously remained unrecognized.

Similar to what applies to other aspects disclosed above, said average expression level may be retrieved from a database.

In an expansion of the method of the seventh aspect, the present invention provides, in a eighth aspect, a method of identifying an agent suitable for AML therapy or as a lead compound for an AML therapeutic, said method comprising the method of the seventh aspect, and bringing a candidate compound into contact with a selected target, wherein modified activity, preferably reduced activity of said target compared to activity of said target in absence of said compound is indicative of said compound being a suitable agent or lead compound.

Reduced activity is preferred in those cases where the candidate therapeutic target exhibits elevated expression. Increased activity upon bringing said candidate compound into contact with said target is preferred in those cases where the candidate target exhibits decreased expression as compared to said average expression level.

In a preferred embodiment - and this applies to all aspects requiring the determining of protein expression levels - said determining is effected by mass spectrometry (MS), Immuno-histochemistry, ELISA, Flow cytometry, antibodies, Western blotting, biosensors, microscopy techniques combined with benchtop fluidics systems such as Akoya's CODEX® instrument, and/or microfluidic immunoassay chips, preferably by MS.

In a ninth aspect, the invention provides a device configured for determining protein expression levels of a set of proteins, wherein said set of proteins comprises or consists of (i) at least three mitochondrial proteins; or (ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, , CSK, MRPL19, NUP88 and nucleoprotein TPR.

Such device, when in use, may employ any technique suitable for determining protein expression levels. As such, one may choose compact and/or rapid implementations which provide for a device which lends itself particularly for point-of-care diagnostics.

In a preferred embodiment, said device comprises a mass spectrometer, wherein preferably said mass spectrometer is configured for selected reaction monitoring (SRM) targeted to said proteins.

In a tenth aspect, the present invention provides a kit comprising or consisting of at least three antibodies, wherein each of said antibodies is specific for one protein selected from a set of proteins, wherein said set of proteins consists of at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

Such kit may comprise further components such as means for rendering binding of an antibody to one of the recited proteins detectable. ELISA is a preferred assay in this respect.

In an eleventh aspect, the present invention provides a computer-implemented method of training a predictor of overall survival or response to chemotherapy of an AML patient and/or of effective treatment of said patient, said method comprising providing a training data set to a machine learning model, wherein said training data set associates protein expression levels as defined in accordance with the first aspect with overall survival, response to chemotherapy and/or effective treatment.

The term "effective treatment" preferably refers to a pharmaceutically active agent or a combination of pharmaceutically active agents which has been observed to be effective in patients with a given protein expression profile. The term "protein expression profile" as used herein designates a set of quantitative protein expression data. Preferred protein sets or subsets are those defined in relation to the first aspect of this invention. A protein expression profile with elevated expression of mitochondrial proteins or subsets thereof as defined herein is associated with inhibitors of mitochondrial function (and preferred implementations thereof as disclosed above) as an effective treatment.

In a preferred embodiment, said machine learning model is selected from gradient-boosted decision trees (see, e.g., Chen, Tianqi, and Carlos Guestrin. "Xgboost: A scalable tree boosting system." Proceedings of the 22nd acm sigkdd international conference on knowledge discovery and data mining. 2016.); survival models (see, e.g. Katzman, Jared L., et al. "DeepSurv: personalized treatment recommender system using a Cox proportional hazards deep neural network." BMC medical research methodology 18.1 (2018): 1-12; Lin, Danyu Y., and Lee-Jen Wei. "The robust inference for the Cox proportional hazards model." Journal of the American statistical Association 84.408 (1989): 1074-1078; and De Iorio, Maria, et al. "Bayesian nonparametric nonproportional hazards survival modeling." Biometrics 65.3 (2009): 762-771.); regularized regression methods such as lasso, elastic net and ridge regression; partial least squares (PLS) regression; support vector machines (SVM); random forest (RF); neural networks; deep learning; logical models; and kernelised bayesian matrix factorization (KBMF).

Related to the first, third, fourth, fifth and eleventh aspect, the present invention provides a data processing device comprising means for carrying out the method of the first, second, third or eighth aspect. Such data processing device may be implemented as a computer, a personal computer, a tablet, a workstation or a smartphone.

Also related to the first, third, fourth, fifth and eleventh aspect, the present invention provides a computer program comprising instructions which, when the program is executed by a computer, cause the computer to carry out the method of the first, second, third or eighth aspect.

Related to the first, third, fourth, fifth and eleventh aspect as well, the present invention provides a computer-readable medium comprising instructions which, when executed by a computer, cause the computer to carry out the method of the first, second, third or eighth aspect.

In a preferred embodiment of all aspects of the invention which recite at least three proteins, said at least three proteins are selected from CYRIB (also known as FAM49B), CSK, MRPL19, NUP88 and nucleoprotein TPR. It is particularly preferred to use a set comprising or consisting of all five of said proteins, i.e., CYRIB, CSK, MRPL19, NUP88 and nucleoprotein TPR.

In preferred embodiments of the preceding aspects, said proteins are detectable by mass spectrometry (MS), Immuno-histochemistry, ELISA, Flow cytometry, antibodies, Western blotting, biosensors, microscopy techniques combined with benchtop fluidics systems such as Akoya's CODEX® instrument, and/or microfluidic immunoassay chips, preferably by MS.

The Figures show:

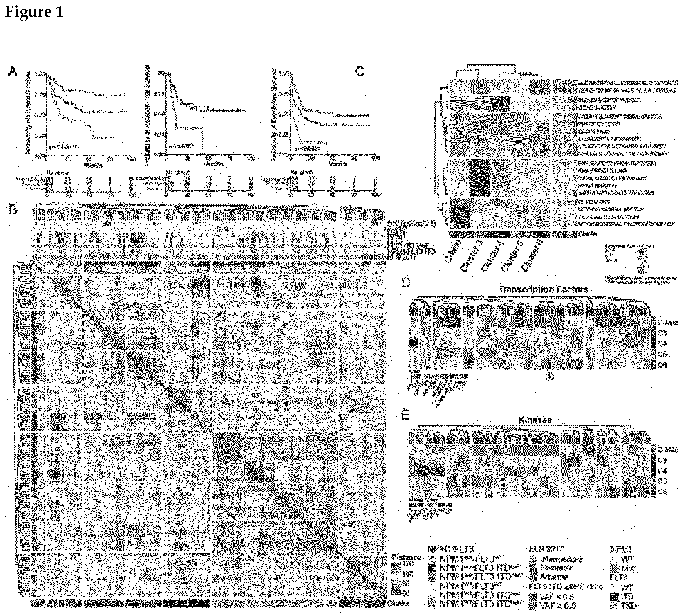

**Figure 1: Proteomic AML subtypes** Mass-spectrometry-based label-free quantification of proteins in bone-marrow-derived AML blasts from 177 untreated AML patients. A Survival probability stratified by ELN2017 classification of cytogenetic risk. Overall, relapse-free and event-free survival are shown for the 177 patients belonging to the discovery cohort. B Unsupervised, hierarchical clustering of patient-patient distances. Rows and columns correspond to patients, with each entry in the heatmap quantifying the distance between two patients. The clustering is used to define six distinct clusters (C1-C6, color-coded). Annotations at the top of the heatmap show patient risk category according to the ELN2017 classification, mutations and cytogenetic aberrations. C Cluster-wise up- and downregulation of gene set variation scores (GSVA) based on differentially regulated Gene Ontology (GO)-pathways (left heatmap; shown as red and blue respectively) Cluster-wise Spearman correlation coefficient of the proteome and the transcriptome for the annotated pathways. Dots mark significantly correlated pathways (right heatmap, $p<0.05$, Benjamini-Hochberg-corrected). D Cluster-wise up- and downregulation of transcription factors; The top annotation color-codes DNA-binding domains. E Cluster-wise up- and downregulation of human kinases. The top annotation color-codes kinase family. Cluster-wise up- and downregulation of human kinases. The top annotation color-codes kinase family.

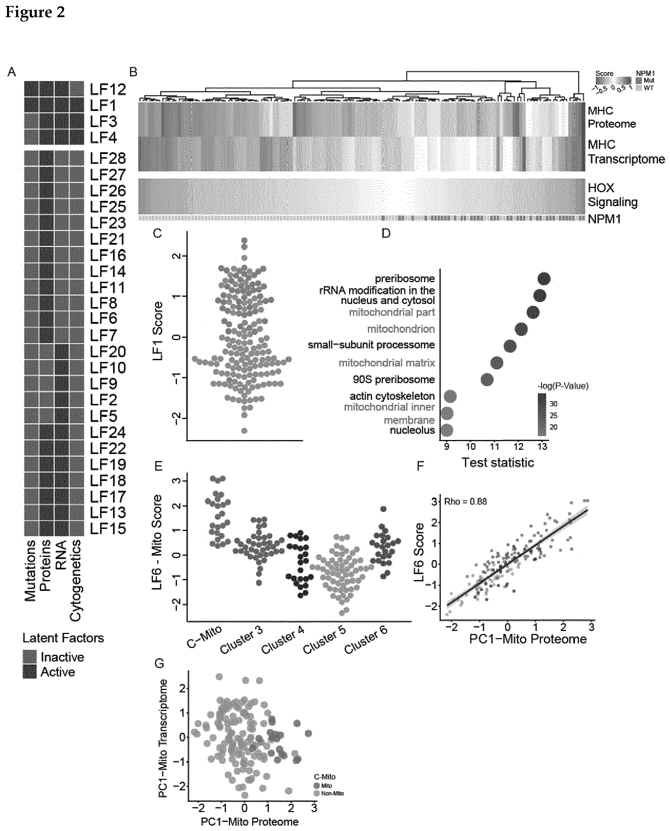

**Figure 2: The proteogenomic characteristics of AML** Multi-omics data analysis integrating proteome, transcriptome, mutation and cytogenetic risk profiles. A 27 factors drive variation in all data layers. Rows represent latent factors; columns represent data modalities. Each square color-codes whether a factor is active in a specific data layer. B Patient-specific enrichment scores for LF1-NPM1/HOX, where each line corresponds to a patient and color encodes the pathway- and patient-specific normalized enrichment score. MHC explains variation in a coordinated fashion for both proteome and transcriptome (top). HOX signaling is enriched in NPM1 mutant patients in the transcriptome (bottom; annotated with NPM1 mutation status). C LF1-NPM1/HOX scores color-coded by NPM1 mutation status. Each symbol corresponds to a patient. D Pathway analysis for factor weights of LF6-mito show that LF6-mito is enriched for pathways related to mitochondrial processes. E LF6-mito scores are shown for each proteomic cluster; LF6-mito scores largely separate patients assigned to C Mito and C5. F Scatter plot of the LF6-mito score (y-axis) versus the first PC of the mitochondrial proteome (x-axis) for each patient. The two factors are strongly correlated (Spearman's rho of 0.88). Proteomic cluster membership is color-coded. G Principal component analyses of the proteome and transcriptome for genes annotated to the mitochondrion only. C-Mito patients can be recovered from the proteome, but not the transcriptome when considering mitochondrial genes only. Each symbol corresponds to a patient; patients assigned to C-Mito are highlighted in red.

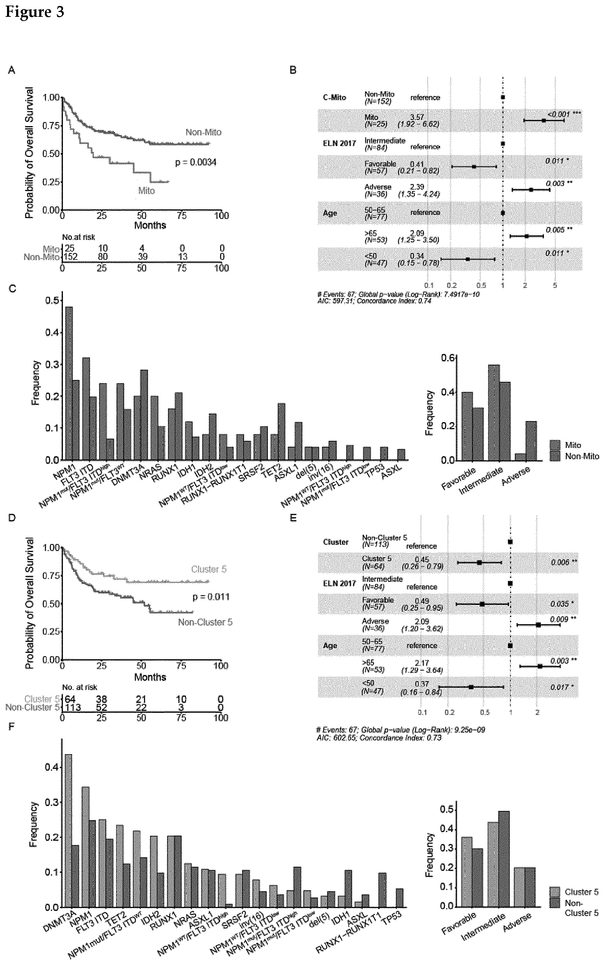

**Figure 3: Relationship between AML proteomic subtypes and survival after intensive induction therapy** A Kaplan-Meier model of overall survival for the Mito and non-Mito patients. B Multivariate analysis of prognostic factors for overall survival including C-Mito. C (Left panel) Relative frequency of cytogenetic and molecular genetic features in the Mito and non-Mito groups. (Right panel) Frequency of ELN cytogenetic risk categories in the Mito and non-Mito groups. D Kaplan-Meier model of overall survival for the C5 and non-C5 patients. E Multivariate analysis of prognostic factors for overall survival including C5. F (Left panel) Relative frequency of cytogenetic and molecular-genetic features in the C5 and non-C5 groups. (Right panel) Frequency of ELN cytogenetic risk categories in the C5 and non-C5 groups.

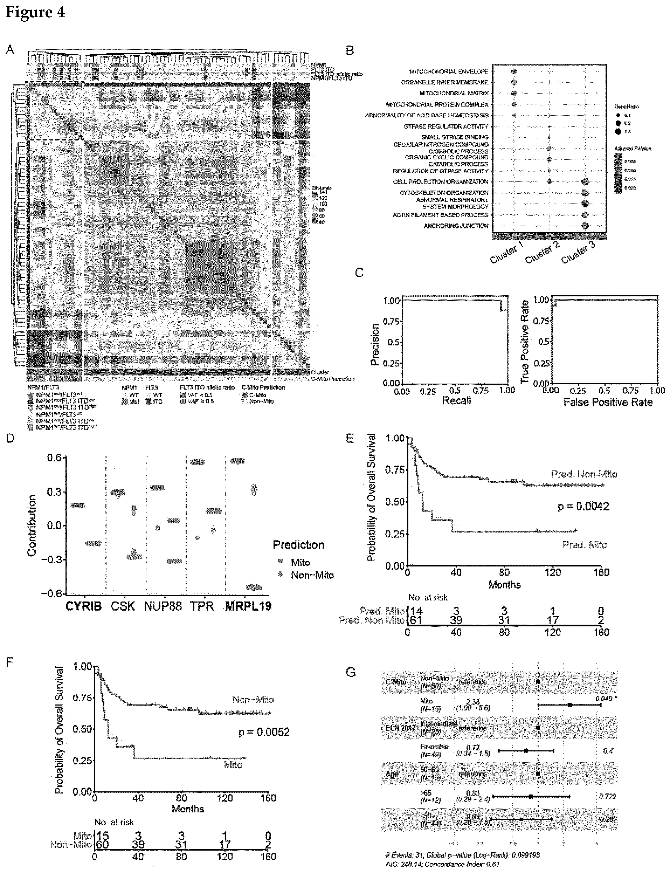

**Figure 4: Validation cohort and Machine learning-based C Mito prediction** Super-SILAC-based quantification of proteins in bone-marrow-derived AML blasts from 75 untreated AML patients (validation cohort). A Unsupervised, hierarchical clustering of patient-patient distances. Rows and columns correspond to patients, with each entry in the heatmap quantifying the distance between two patients. The clustering is used to define three distinct clusters (C1-C3; color-coded). Annotations at the top of the heatmap show NPM1 mutation status, FLT3/ITD status, FLT3/ITD allelic ratio and NPM1/FLT3 status . Annotated at the bottom is a C-Mito class membership predicted by a classifier trained on the discovery cohort (predicted C-Mito in red). B Characterization of the three proteomic clusters using a pathway analysis of differentially expressed proteins between each cluster and the remaining patients. Mitochondrial terms were the distinct features characterizing cluster 1. C Precision versus recall and receiver operating characteristic (ROC) curve of the C-Mito classifier, which was trained on the discovery cohort and tested on the validation cohort. Metrics were computed on the basis of unsupervised clustering of the validation cohort (C1 patients were considered as Mito). Areas under both curves close to 1 indicate a high concordance between the unsupervised analysis of the validation cohort only and classification supervised by the discovery cohort. D Beeswarm plot of the contributions of the 5 most relevant proteins to the classification of patients to C-Mito in the validation cohort. Each symbol represents a patient in the validation cohort and patients classified as C-Mito are shown in red, patients classified as non-mito in grey. Bold print marks mitochondrial proteins. E Kaplan-Meier model of overall survival for the patients of the validation cohort predicted to be Mito and non-Mito by using the classifier trained on the discovery cohort. Patients classified as C-Mito had significantly shorter overall survival (OS). F Kaplan-Meier model of overall survival for the Mito and non-Mito patients of the validation cohort based on hierarchical clustering; C-Mito patients again had a significantly shorter OS. G Multivariate analysis of prognostic factors for overall survival including C-Mito (hierarchical clustering-based) of the validation cohort.

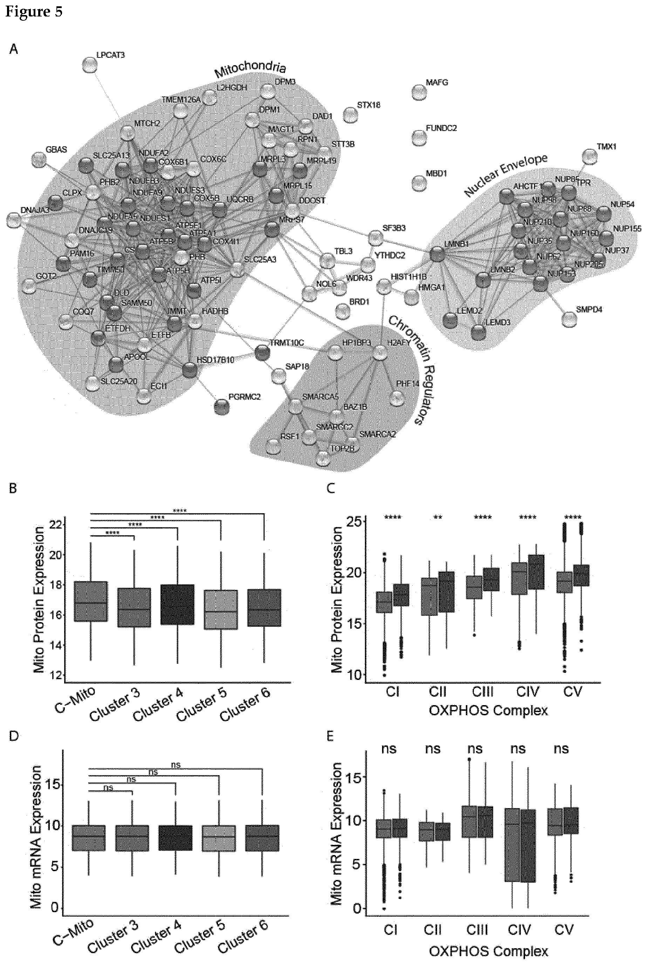

**Figure 5: The Mito-AML-defining proteomic network** A StringDB-network of the top 100 differentially (FDR<0.01) upregulated proteins in C Mito versus non-Mito patients. Each node represents a protein, and edges indicate interaction type. Three major protein hubs emerge, corresponding to mitochondria, chromatin regulators and nuclear envelope respectively. Node colors mark enriched pathway terms (red = mitochondrial protein complex and cellular respiration, yellow = chromatin remodeling, violet = nuclear envelope). B Expression of mitochondrial proteins (log2-intensity) stratified by proteomic cluster. Proteomic cluster C-Mito has significantly higher expression of mitochondrial proteins than Cluster 3 to Cluster 6 (Wilcoxon rank sum test). Mitochondrial proteins were selected as those annotated to the term Mitochondrion in GOcc (GO:0005739). C Expression of OXPHOS complex subunits in the proteome (log2-intensity) stratified by C-Mito. Patients in C-Mito had a significantly higher expression of OXPHOS complex subunits in the proteome than the remaining patients. D mRNA expression of mitochondrial genes (GOcc mitochondrion), stratified by proteomic cluster. No significant differential expression was found (Wilcoxon rank sum test). E mRNA expression of OXPHOS complex subunits, stratified by C-Mito. In the transcriptome, expression of OXPHOS complex subunits is not significantly different between C-Mito and non-Mito patients (Wilcoxon Rank Sum test).

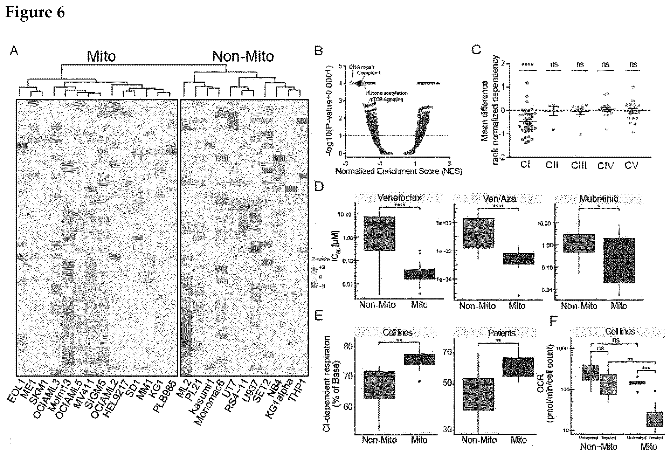

**Figure 6: Metabolic wiring and therapeutically relevant vulnerabilities of Mito-AML** A Hierarchical clustering of leukemia cell lines based on expression of 101 OXPHOS related proteins. Columns correspond to cell lines, rows correspond to proteins. The clustering is used to split leukemia cell lines into two groups (Mito and Non-Mito). B Volcano Plots plot depicting the gene-set enrichment for the genome-wide differential CERES dependency score in Mito vs non-Mito cell lines available in the CRISPR (Avana) 21Q2 data. Significance: abs(NES) > 1.3 and, P-value <0.1. Complex I gene sets are highlighted red. C Scatter mean +/-SD dot plot depicting mean difference rank normalized CERES dependency scores in Mito vs. non-Mito cell lines across OXPHOS complexes I-V. One-sample t-test. CRISPR (Avana) 21Q2 dependency data. D The half-maximum inhibitory concentration (IC50) of Mito AML cell lines (n=9) and non-Mito AML cell lines (n=10) is shown. Mito AML cell lines have increased sensitivity towards OXPHOS targeting drugs (Wilcoxon rank sum test). E Respiratory chain complex I dependency is quantified in AML cell lines (n Mito = 8, n non-Mito = 9) and primary patient samples (n Mito = 9, n non-Mito = 11, all from the discovery cohort) using a Seahorse 96 extracellular flux analyzer. Both for patient samples and cell lines, Mito samples exhibited significantly stronger Complex I dependency (p<0.01 for patient samples and cell lines; Wilcoxon rank sum test). Complex I dependency is measured as a percentage of basal oxygen consumption rate after inhibition with the complex I inhibitor rotenone. F Oxygen consumption rate in cell lines measured after 24 h treatment with 2 $\mu$M venetoclax shows a marked decrease in Mito AML cell lines (n=8; p<0.001, Wilcoxon rank sum test) while no significant decrease is seen in non-Mito cell lines (n=8, p = 0.13, Wilcoxon rank sum test). The decrease is significantly greater in Mito compared to non-Mito cell lines (p<0.01, Wilcoxon rank sum test).

The Examples illustrate the invention.

**Example 1**

Materials and Methods

*Ethics statement*

Bone marrow biopsies from AML patients were obtained and collected pre- and posttreatment. All patients gave informed consent according to the Declaration of Helsinki to participate in the collection of samples. The use of bone marrow aspirates was approved by the Ethics Committee of University Hospital Frankfurt (approval No. SHN-12-2016) and the study alliance leukemia (SAL, approval No. EK98032010).

*Cell Lines*

Human AML cell lines including THP-1 (DSMZ no. ACC16; FAB M6, Sex male), OCI-AML2 (DSMZ No. ACC 99; FAB M4, Sex male), OCI-AML3 (DSMZ No. ACC 582; FAB M4, Sex male), Molm13 (DSMZ No. ACC 554; FAB M5a, Sex male), PL-21 (DSMZ No. ACC 536; FAB M3, Sex male), HL-60 (DSMZ No. ACC 3; FAB M2, Sex female), MV4-11 (DSMZ No. ACC 102; FAB M5, Sex male), SIG-M5 (DSMZ No. ACC 468; FAB M5a, Sex male), ML2 (DSMZ No. ACC 15; FAB M4, Sex male), NB4 (DSMZ No. ACC 207; FAB M3, Sex female), KG1 (DSMZ No. ACC 14; FAB not indicated, Sex male), KG-1a (DSMZ No. ACC 421; FAB not indicated, Sex male), U-937 (DSMZ No. ACC 5, FAB not indicated, Sex male), Kasumi-1 (DSMZ No. ACC 220, FAB M2, Sex male), EOL-1 (DSMZ No. ACC 386, FAB not indicated, Sex male), OCI-AML5 (DSMZ No. ACC 247, FAB M4, Sex male) , UT-7 (DSMZ No. ACC 137, FAB M7, Sex male), SET-2 (DSMZ No. ACC 608, FAB no indicated, Sex female), MonoMac6 (DSMZ No. ACC 124; FAB M5, Sex male) and HEL (DSMZ No. ACC 11; FAB M6, Sex male) were obtained from DSMZ (Deutsche Sammlung von Mikroorganismen und Zellkulturen GmbH). THP-1, OCI-AML2, OCI-AML3, Molm13, PL-21, HL-60, MV4-11, SIG-M5, ML2, NB4, KG1, KG-1a, U-937, EOL-1, SET-2, MonoMac6 and HEL were cultured in RPMI 1640 (ThermoFisher, Cat. No. 11875093), supplemented with 20% FBS, 4 mM L-glutamine, 100 IU/ml penicillin and 100 mg/ml streptomycin. Kasumi-1 and OCI-AML5 were cultured in Advanced RPMI (ThermoFisher, Cat. No. 12633012) supplemented with 5% FBS, 4 mM L-glutamine, 100 IU/ml penicillin and 100 mg/ml streptomycin. UT-7 were cultured in IMDM (ThermoFisher, Cat. No. 12440061) supplemented with 10% FBS, 4 mM L-glutamine, 100 IU/ml penicillin, 100 mg/ml streptomycin and 5 ng/ml GM-CSF (Pebrotech, Cat. No. 300-03). All cell lines were kept at 37 °C in a humidified 5% $CO_2$ incubator. Cells were routinely tested for mycoplasma contamination (LT07-710, Lonza) and authenticated by short-tandem-repeat profiling, as reported (Capes-Davis et al., 2013).
ABT-199/Venetoclax was purchased from Selleckchem (Cat. No. S8048), TAK 165/Mubritinib was purchased from Selleckchem (Cat. No. S2216), 5-Azacytidine was purchased from Selleckchem (Cat. No. S1782).

*Primary Cells*

Patient-derived AML blasts (2-5 × 106) were cultured in X-vivo 10 medium (Lonza, Cat. No. BE04-380Q) supplemented with 10% HyClone FCS (Perbio, Cat. No. SH30071.03HI), 4 mM L-glutamine, 25 ng/ml recombinant human TPO (300-18), 50 ng/ml recombinant human SCF (300-07), 50 ng/ml recombinant human Flt3-ligand (300-19) and 20 ng/ml recombinant human IL3 (200-03, all from Pebrotech).

*Patient cohorts*

We obtained pre-therapeutic bone-marrow samples and clinical data from 252 AML patients aged from 18 to 79 years. Patients of the discovery cohort (n = 177) were admitted to the University Hospital Frankfurt between 2012 and 2019 and treated for newly diagnosed acute myeloid leukemia with at least one cycle of intensive induction therapy (Ara-C (100 mg/m2) for 7 d and daunorubicin (60 mg/m2) for 3 d ("7+3 regimen")). Bone marrow biopsies and aspirates were obtained according to the hospital's standard procedure. Mononuclear cells from pretherapeutic AML bone marrow were purified by Ficoll-Hypaque gradient centrifugation (Pancoll human, Pan Biotech) and cryopreserved at the institutional biobank (UCT Frankfurt, Germany). Patient samples with AML blast purity >75% (as assessed by flow cytometry/ CD45dim/SSClow blast gate) after Ficoll purification were included in this study. The median blast count after Ficoll enrichment was 84%. At the University Hospital Frankfurt patients are routinely advised to undergo a bone marrow biopsy at diagnosis, at day-15 of the induction therapy and after completion of induction therapy for response assessment.

Eligible patients with day-15 blasts >5%, no complete remission after induction therapy, adverse risk cytogenetics and intermediate risk cytogenetics with matching donor undergo allogeneic stem cell transplantation. Remission criteria and cytogenetic risk groups were assessed according to the ELN guidelines (Döhner et al., 2017).

Purified mononuclear cells (purity >75%) and clinical data from patients of the validation cohort (n = 75) were obtained from the biobank of the Study Alliance Leukemia (SAL, Dresden, Germany). Followed by a density gradient centrifugation step based on Biocoll (Biochrom), isolated mononuclear cells were washed twice and resuspended in cryogenic medium. Patients included in this cohort were treated for newly diagnosed AML between 1997 and 2009 as explained above. All patients consented to the scientific analysis of their data and biomaterial that was obtained for diagnostic purposes. First complete remission (CR1) was defined as bone marrow blast count < 5% and absence of circulating blasts and blasts with Auer rods 45 days after the last induction cycle with or without residual neutropenia (<$1.0 \times 10^9$/L [1000/$\mu$L]) or thrombocytopenia (<$100 \times 10^9$/L [100 000/$\mu$L]). Relapse-free survival (RFS) and remission duration were calculated only for patients that achieved complete remission (CR).

Demographic and diagnostic variables were ascertained at the time of diagnosis by the University Hospital Frankfurt and additional trial centers of the SAL. The variables collected included: Age at diagnosis, gender, karyotype, cytogenetic risk, genetic risk according to the European Leukemia Net (ELN) recommendations and AML type (AML: de novo AML, sAML: secondary AML, tAML: therapy-related AML for patients with prior chemotherapy). Peripheral blood counts included white blood cell (WBC) counts, platelet counts, hemoglobin levels (Hb) and bone marrow and peripheral blood blasts.

**Table 1: Clinical Characteristics of the AML Patients**

|  | Discovery Cohort (N = 177) | Validation Cohort (N = 75) |
|---|---|---|
| Age at first diagnosis (years) |  |  |
| Median | 58 | 46 |
| Range | 18.6-79.0 | 18.0-78.0 |
| Female Sex - no. (%) | 72 (40.7) | 43 (45.3) |
| European LeukemiaNet classification - no./total no. (%) |  |  |
| Favorable | 57/177 (32.2) | 49/75 (65.3) |
| Intermediate | 84/177 (47.5) | 25/75 (33.3) |
| Adverse | 36/177 (20.3) | 1/75 (1.3) |
| French-American-British (FAB) classification - no./total no. (%) |  |  |
| M0 | 8/177 (4.5) | 0/75 (0) |
| M1 | 32/177 (18.1) | 11/75 (14.7) |
| M2 | 66/177 (37.3) | 25/75 (33.3) |
| M4 | 46/177 (26.0) | 9/75 (12.0) |
| M4Eo | 11/177 (6.2) | 20/75 (26.7) |
| M5 | 4/177 (2.3) | 0/75 (0) |
| M5a | 2/177 (1.1) | 2/75 (2.7) |
| M5b | 7/177 (4.0) | 1/75 (1.3) |
| M6 | 1/177 (0.6) | 0/75 (0) |
| Not classified |  | 7/75 (9.3) |
| Precursor - no./total no. (%) |  |  |
| Secondary AML | 29/177 (16.4) | 2 (2.7) |
| Therapy related AML | 7/177 (4.0) | 3 (4.0) |
| White blood count at first Diagnosis - mg/dL |  |  |
| Median | 10,000 | 30,000 |
| Range | 400 - 289,000 | 2,000 - 287,000 |
| Second induction cycle - no./total no. (%) | 126/177 (71.2) | 63/75 (84.0) |
| Allogeneic stem cell transplantation - no./total no. (%) | 104/177 (58.8) | 21/75 (28.0) |

*Proteomics*

Sample preparation for the discovery cohort

Enriched AML blasts (5x106 viable cells per sample) were directly lysed (without any prior ex vivo culture) in 1% SDC buffer (1% SDC, 100mM Tris pH8.0, 10mM CAA, 40mM TCEP pH7.0), boiled at 95°C for 5 minutes and cooled on ice for 20 mins, followed by 10 cycles of sonication for 10 minutes (Branson probe sonifer output 3-4, 50% duty cycle, 10x 30 sec) and finally heated again at 95°C for 5 minutes. Notably, cell lysis using this buffer and immediate heating at 95° for 5 minutes achieves inactivation of all proteases in the sample, including neutral serine proteases as they are heat-labile proteases. Furthermore, the presence of TCEP (protein disulfide bond reducing agent) and chloro-acetamide (protein thiol groups alkylation) leads to protein unfolding and irreversible enzyme inactivation. Protein concentration was estimated by BCA assay and 20 μg from each sample were digested with LysC (1:100) and Trypsin (1:100) overnight using a thermoshaker (1200 rpm at 37°C). To stop the digestion, 5x volume isopropanol/1% TFA was added and vortexed vigorously. The peptides were desalted on SDB-RPS StageTips, washed once with isopropanol/1%TFA and once with 0.2% TFA. The peptides were eluted with 60μl of elution buffer (80%, 1.25% NH4OH). The dried eluates were resuspended in MS loading buffer (3%ACN, 0.3% TFA) and stored at -20°C until MS measurement. This procedure yielded 90% intact tryptic peptides which is in the range of previously published studies (Jayavelu AK et al., 2020).

Nano-flow LC-MS/MS proteome measurements

All the samples were analyzed on nanoflow HPLC coupled to a high-resolution mass spectrometer. Briefly, peptides were loaded onto 50 cm reversed phase columns, packed in-house (75 μm inner diameter, ReproSil-Pur C18-AQ 1.9 μm resin Dr. Maisch GmbH). The column temperature was controlled at 60°C using a homemade column oven. An Easy-nLC 1200 system was connected to the mass spectrometer (Q Exactive HF-X, Thermo Fisher Scientific) with a nano spray ion source, and binary buffer system, consisting of buffer A (0.1% formic acid) and buffer B (0.1% FA in 80% ACN), was utilized for low pH peptide separation at a flow rate of 300 nl/min. For individual measurements, 400ng of peptide material was loaded and eluted over 120 min for patient samples or 140 min for cell lines. For a 120 min sample run, the gradient was stepwise increased from 5% to 30% buffer B over 105 min and stepped up to 60% over 5 min followed by wash in 95% over 10 min before re-equilibration in buffer A.

Label-free LC-MS/MS data acquisition

For experiments involving data-dependent data acquisition (DDA), the Thermo Xcalibur (3.0.63) software was used for the Q Exactive HF-X instrument. The Q Exactive HF-X was operated in Top15 mode with a full scan range of 300-1650 m/z at a resolution of R=60,000 at 200m/z. The automatic gain control (AGC) was set to 3e6 at a maximum injection time of 20 s. Precursor ion selection width was kept at 1.4 m/z and fragmentation was achieved by higher-energy collisional dissociation (HCD) (target 1e5 ions, maximum filling time 120ms, isolation window 1.6 m/z, and normalized collision energy 27%). Fragment ion scans were recorded at a resolution of 15,000, an AGC of 1e5 and a maximum fill time of 60 ms. Dynamic exclusion was enabled and set to 30 s. Charge exclusion (unassigned, 1,6, -8 & >8) was enabled.
For experiments involving data-independent data acquisition (DIA), the Thermo Xcalibur (3.0.63) software was used for the Q Exactive HF-X instrument. The Q Exactive HF-X was operated with scan range set to 350-1450 m/z at a resolution of 60,000. The AGC was set to 3e6 at a maximum injection time of 54 ms. HCD (NCD 27%) was used for precursor fragmentation and fragment ions were analyzed in 32 DIA windows at a resolution of 30,000, while the AGC was kept at 3e6.

Super-SILAC-based LC-MS/MS

Primary AML samples of the validation cohort were washed twice with PBS followed by cell number counting. One million cells were lysed in 30 μl of NP-40 buffer (50 mM TRIS-HCl, pH 7.5, 150 mM NaCl, 0.5% NP-40, 5 mM NaF, 1 mM Na3VO4, protease inhibitor cocktail) and protein concentration was determined using a BCA assay. Subsequently, 40 μg of protein from each sample were mixed with 40 μg of protein from a SILAC reference standard. The SILAC standard consisted of equal protein amounts from 3 different AML cell lines (KG1, NB4, MV4-11), which had been subjected to SILAC with 13C615N4 L-arginine and 13C615N2 L-lysine before protein extraction with NP-40 buffer. The mixed sample was incubated with LDS sample buffer and reducing agent (NuPAGE, Thermo Fisher Scientific) according to the instructions of the manufacturer and subjected to polyacrylamide gel electrophoresis. The separated proteins were stained with Coomassie Brilliant Blue and the whole gel lane was excised as 23 individual bands, followed by in-gel protein digestion with trypsin.

The peptide mixtures were analyzed on a Q Exactive Orbitrap mass spectrometer (Thermo Fisher Scientifc) coupled to an EASY-nLC 1200 HPLC system (Thermo Fisher Scientific) via a nano-ESI source (Nanospray Flex, Thermo Fisher Scientific). The peptides were first trapped on a precolumn and then separated on an analytical column (ReproSil-Pur 120 C18-AQ, 3 $\mu$m; Dr. Maisch GmbH; 200 x 0.075 mm; self-packed) with a 90 min linear gradient of 2-40% solvent B (80% ACN, 0.08% FA) versus solvent A (0.1% FA). Eluting peptides were analyzed by data-dependent acquisition using a top 15 MS/MS method with a survey scan resolution setting of 70,000 FWHM and an MS/MS resolution setting of 17,500 FWHM at m/z 200, respectively. The 15 most abundant ions within the m/z 350-1600 range were selected for HCD with an NCE setting of 28% and an isolation width of m/z 2.0, excluding singly charged ions and charge states >4. AGC target values and maximum ion injection times for MS and MS/MS were set to 1x106 in 60 ms and 2$\times$105 in 60 ms, respectively. Selected precursor mass-to-charge ratio values were dynamically excluded from fragmentation for 15 s.

DDA data analysis

MS raw files were processed in MaxQuant (Tyanova et al., 2016a) (version 1.5.5.2) open software environment utilizing Andromeda search engine against the human UniProt reference proteome database (version 2014 containing 88993 entries) including variable modifications oxidation & acetylation at N-terminus and Carbamidomethyl cysteine as fixed modification. Enzyme digestion specificity was set to Trypsin and maximum two missed cleavages were allowed. Parent peptide mass tolerance and fragment peptide mass tolerance up to 4.5-20ppm were allowed. A cut-off 1% FRD was applied first at the peptide level and second at the protein level. A minimum of 7 amino acids was required for peptide identification. Each Super-SILAC-labeled AML sample was measured in 23 fractions resulting in 23 raw files and these were combined for each individual patient sample. When sequence spectra were absent in specific samples, peptide identification and quantification in the remaining samples was facilitated through enabling the 'match between runs' option in MaxQuant. If proteins could not be distinguished on the basis of unique peptides, they were merged by MaxQuant into one protein group. For the relative quantification of SILAC pairs the minimum ratio count in MaxQuant was set to 1 as default setting. Label-free protein quantification was performed using the MaxLFQ (Cox et al., 2014) algorithm and 'match-between-runs' was enabled. For stringent filtering a minimum peptide ratio count of 2 and at least 1 razor peptide for protein identification were enabled. Further protein groups identified as common contaminants, reverse hits and site-modifications were filtered out.

DIA data analysis

DIA raw files were analyzed using Spectronaut Pulsar (Biognosys, Cat. No. Sw-3001, version 13.10.191212) using the default settings for targeted DIA analysis. A project-specific spectral library from cell lines encompassing 170,667 precursors, corresponding to 8198 protein groups or patient samples encompassing 65,914 precursors, corresponding to 6551 protein groups was generated utilizing pulser against the human Uniprot reference proteome database (version August 2018, containing 21,039 entries) and used. Data export was filtered by 'No Decoy' and 'Quantification Data Filtering' for peptide and protein quantifications.

Proteome data imputation

We performed the majority of bioinformatic analyses on unimputed data - this includes clustering, PCA analyses, correlation analyses and MOFA. For PCA and MOFA probabilistic implementations were used. Where imputed data is required, the expression data was first filtered to 80% valid values. We then performed data imputation using the in house open source Perseus environment (version1.5.2.11) (R Core Team, 2020; Tyanova et al., 2016b) with default parameters (Gaussian imputation with parameters width=0.5 and downshift=1.5) - an approach that is widely used for proteomics data imputation. In brief, Perseus uses a normal distribution to model signals for low abundant proteins under the assumption that the low signals are due to the detection limit of the MS measurement. A transformed normal distribution with mean and standard deviation estimated from the samples from the dataset is used to represent MNAR values.

*Genomics*

mRNA extraction and whole transcriptome sequencing

For transcriptome analysis the TruSeq Total Stranded RNA kit was used, starting with 250ng of total RNA, to generate RNA libraries following the manufacturer's recommendations (Illumina, San Diego, CA, USA). 100bp paired-end reads were sequenced on the NovaSeq 6000 (Illumina) with a median of 57 mio. reads per sample.

RNA Data Analysis

Data quality control was performed with FastQC v0.11.9. Reads were aligned to the human reference genome (Ensembl GRCh38 release 82) using STAR v2.6.1. Gene count tables were generated while mapping, using Gencode v31 annotations. All downstream analyses were carried out using R v4.0 and BioConductor v3.12 (Huber et al., 2015; R Core Team, 2020). Size-factor based normalization was performed using DESeq2 v1.28.1(Love et al., 2014).

mRNA extraction and transcriptome sequencing of LSC subpopulations Flow cytometry

For RNAseq experiments primary samples were FACS sorted using BD FACS Aria Fusion (BD Biosciences). Fluorescence conjugated antibodies targeting human CD3, CD20 (Biolegend) and CD235a (BD Biosciences) were used for lineage positive cells exclusion. Antibodies targeting human CD45, CD38, CD34 (BD Biosciences), GPR56 (Biolegend) were used together with simultaneous staining of all NKG2D ligands using a recombinant biotinylated NKG2D-Fc chimera as described in (Paczulla et al., 2019), to enrich for stem cells. Detailed gating strategy will be described in Donato, Correia, Andresen and Trumpp et al., (manuscript in preparation). 7-AAD (BD Biosciences) was used for dead cell exclusion.

RNA extraction

Between 1x103 to 50x103 cells from each subpopulation were directly sorted into RNA lysis buffer (Arcturus PicoPure RNA Isolation Kit, Life Technologies, Invitrogen) and RNA was isolated according to the instructions.

RNAseq library preparation

cDNA was generated as in (Picelli et al., 2014) with minor changes. Briefly, cDNA was produced using SMARTScribe (Takara) and amplified using KAPA Mix (Roche). Amplified DNA was sonicated using Covaris E220 and libraries were generated using NEBNext Ultra II DNA Library Prep Kit for Illumina (New England Biolabs). Libraries were sequenced pair- end with read length of 75 bp using a NextSeq550 High Output (Illumina).

Targeted DNA panel sequencing

Targeted DNA panel sequencing was performed at the Genomics core facility, DKTK/DKFZ, Frankfurt, Germany, the Laboratory for Molecular Hematology at the University Hospital Carl Gustav Carus, Dresden, Germany and at MLL Dx GmbH, Munich, Germany.

Workflow at MLL laboratory, Munich, Germany

Targeted sequencing was performed using the Nextera DNA Flex library preparation kit, starting with 100ng of genomic DNA (Illumina, San Diego, CA, USA). The target regions were enriched by a custom xGen Lockdown panel using a hybridization capture workflow (IDT Integrated DNA Technologies, Coralville, IA, USA). All libraries were sequenced with 100bp paired-end reads on a NovaSeq6000 (Illumina) with a mean coverage of 3206x. Somatic variant calling was performed with Pisces and a sensitivity cut off of 2%. Large deletions and medium-sized insertions, as they are for example found in CALR and FLT3, were called with Pindel. Variant annotation considered the publicly available data bases Cosmic (v91), ClinVar (2020-03), gnomAd (non-cancer, v2.1.1), dbNSFP (v3.5) and UMD TP53 (2017_R2). Variants that are described as somatic, protein truncating or affecting splice sites were considered as mutations while variants with no or discrepant data base information were considered as variant of uncertain significance.

Workflow at DKFZ Genomics Core Facility, Heidelberg/, Frankfurt, Germany

Library Preparation and sequencing

Libraries were prepared from 40 ng DNA using the QIASeq Human Myeloid Neoplasms Panel (Qiagen) according to the manufacturer's protocol. Samples were tagged with the QIAseq 96-Unique Dual Index Set A for Illumina platforms (Qiagen) to yield unique combinations of i5 and i7 barcodes for each sample. Sample fragment size distribution and concentration was estimated using the Agilent High Sensitivity DNA kit on a 2100 Bioanalyzer (Agilent). Samples were pooled in an equimolar fashion, denatured, and diluted to 1.5 pM according to Illumina's recommendations. The diluted library was sequenced on a NextSeq 500 benchtop sequencer (Illumina) using NextSeq High Output cartridges. Demultiplexing was performed using the BaseSpace cloud platform (Illumina).

Workflow at the Laboratory for Molecular Hematology, University Hospital Carl Gustav Carus, Dresden, Germany

Profiling of co-mutations was done by targeted resequencing using the TruSight Myeloid assay (Illumina, Chesterford, UK) covering 54 genes recurrently mutated in AML: BCOR, BCORL1, CDKN2A, CEBPA, CUX1, DNMT3A, ETV6, EZH2, IKZF1, KDM6A, PHF6, RAD21, RUNX1, STAG2, ZRSR2, ABL1, ASXL1, ATRX, BRAF, CALR, CBL, CBLB, CBLC, CDKN2A, CSF3R, FBXW7, FLT3, GATA1, GATA2, GNAS, HRAS, IDH1, IDH2, JAK2, JAK3, KIT, KRAS, MLL, MPL, MYD88, NOTCH1, NPM1, NRAS, PDGFRA, PTEN, PTPN11, SETBP1, SF3B1, SMC1A, SMC3, SRSF2, TET2, TP53, U2AF1 and WT1. For each reaction, 50 ng of genomic DNA was used. Library preparation was done as recommended by the manufacturer (TruSight Myeloid Sequencing Panel Reference Guide 15054779 v02, Illumina). Samples were sequenced paired-end (150 bp PE) on NextSeq- (Illumina) or (300 bp PE) MiSeq-NGS platforms, with a median coverage of 3076 reads, range 824-30565. Sequence data alignment of demultiplexed FastQ files, variant calling and filtering was done using the Sequence Pilot software package (JSI medical systems GmbH, Ettenheim, Germany) with default settings and a 5% variant allele frequency (VAF) mutation calling cut-off. Human genome build HG19 was used as reference genome for mapping algorithms.

*Cytogenetics*

Karyotypes were ascertained in accordance with the International System for Human Cytogenetic Nomenclature from diagnostic assessment.

*Mutation Data Analysis*

Raw reads (FASTQ) files were imported into the CLC Genomics Workbench (version 12, Qiagen) and analyzed using a modified Myeloid Neoplasms Panel (DHS-003Z) workflow. In brief, the raw reads were mapped to the hg19 draft of the human genome and duplicate reads were merged based on the similarity of the incorporated unique molecular identifiers (UMIs), which allowed for accurate identification of variants at low allele frequencies. Variants were called using the proprietary variant caller included in the CLC Genomics Workbench. Called variants were annotated (dbSNP, COSMIC, ClinVar, and gnomAD). All variants appearing in more than 50% of the analyzed samples were considered as technical artefacts and filtered out. Additionally, variants with a mutant allele frequency below 5% or a population frequency about 0.1% were remove from the dataset. Variants previously reported in COSMIC as somatic and variants annotated as protein truncating, frame shift or affecting a splice site were considered as clinically relevant.

*Cell viability assay*

The viability of AML cell lines that were treated with various drug concentrations was determined by the 3-(4,5-dimethylthiazol-2-yl)- 2,5-diphenyltetrazolium bromide (MTT) dye reduction assay after 96 h of incubation, as described previously. IC50 values were determined using the R package "drc" v3.0-1 (Ritz et al., 2015). For venetoclax, a starting concentration of 10 $\mu$M and a 1:4 dilution was used. For venetoclax + azacytidine, a starting concentration of 1 $\mu$M azacytidine and 1 $\mu$M venetoclax was used with a 1:4 dilution for venetoclax. For mubritinib, a starting concentration of 40 $\mu$M was used with a 1:4 dilution.

*Immunohistochemistry*

Immunohistochemical stainings were performed as previously described (Hartmann et al., 2011). In brief, slides were deparaffinized and after heat induced antigen retrieval at pH9, slides were stained with the anti-Ki67 antibody (clone MIB-1, Agilent-DAKO) on an Autostainer (Agilent-DAKO). The Envision-Flex Kit (Agilent-DAKO) was used for detection. Slides were assessed for the number of positively stained AML blasts by an experienced hematopathologist who was blinded to the clinical history of the patients and their therapeutic response.

*Seahorse analysis*

25.000 AML cells (cell lines or patient samples) were seeded on Seahorse 96-well cell culture plates in RPMI (+P/S, +L-Glut, +20% FCS) and the oxygen consumption rate (OCR) was measured using a Seahorse 96 extracellular flux analyzer (Agilent, Waldborn, Germany). To analyze the dependency of mitochondrial respiration on complex I, 500 nM Rotenone (Cayman Chemicals, Michigan, US) was injected after measuring basal respiration. To calculate the complex I dependency, the basal respiration was set to 100%. Afterwards the difference between basal OCR and OCR after Rotenone was calculated. Effects of Venetoclax on mitochondrial respiration was performed by incubating AML cell lines for 24 h with 1 $\mu$M Venetoclax and measuring respiration of 25.000 cells.

*CRISPR-Cas9 Screening*

The analysis was performed on the 21Q2 version of the CRISPR-Cas9 gene effect level data for 859 cancer cell lines including 23 AML, processed on the Avana library (Doench et al., 2016; Sanjana et al., 2014) with the CERES algorithm (Meyers et al., 2017). The Avana library contains 73,372 guides for 18,119 genes, with an average of 4 guides per gene. Out of the 23 AML cell lines available in the CRISPR (Avana) 21Q2 data, ten cell lines were experimentally annotated in this study for the "Mito" status: six cell lines as "Mito-high" (EOL1, HEL9217, MOLM13, MV411, OCIAML2, OCIAML3) and four cell lines as "Mito-low" (KASUMI1, NB4, THP1, U937). The CRISPR (Avana) dependency data are publicly available at the DepMap portal (https://depmap.org/portal/download/). Initially, cancer cell lines were transduced with Cas9 using a lentiviral system. Cell lines that met criteria, including acceptable Cas9 activity measuring ability to knockout transduced GFP, appropriate growth properties and other parameters, were then screened with the Avana library. A pool of guides was transduced into a population of cells. The cells were cultured for 21 days in vitro, and at the end of the assay, barcodes for each guide were sequenced for each cell line in replicate. The sgRNA read count data were deconvoluted from sequence reads by using the PoolQ tool available from the Genetic Perturbation Platform at the Broad Institute https://portals.broadinstitute.org/gpp/public/software/poolq.

Several quality control pre-processing steps were performed to remove samples with poor replicate reproducibility, as well as guides that have low representation in the initial plasmid pool, as described in (Dempster et al., 2019).

The raw readcounts were summed up by replicate and guide and the log2-fold-change from pDNA counts for each replicate was computed. The sgRNAs with suspected off-target activity and the guides with pDNA counts less than one millionth of the pDNA pool were removed. The replicates that failed fingerprinting and the replicates with less than 15 million reads were removed and then the replicate read counts were scaled to 1 million total reads per replicate. The replicates with the null-normalized mean difference (NNMD) greater then -1.0 were filtered out. The replicates that did not have a Pearson coefficient > .61 with at least one other replicate for the line when looking at genes with the highest variance (top 3%) in gene effect across cell lines were removed. Then NNMD was computed again for each cell line after averaging remaining replicates, and the cell lines with NNMD > -1.0 were filtered out.

For quality control and normalization, exogenously defined nonessential genes (Hart et al., 2014) were used as negative controls and common essential genes (Blomen et al., 2015; Hart et al., 2015) were used as positive controls.

The gene level dependency scores were inferred by running the computational tool CERES (Meyers et al., 2017). CERES was developed to computationally correct the copy-number effect and to infer true underlying effect of gene knockout as described in (Meyers et al., 2017). The CERES algorithm models the observed normalized log-fold change for each sgRNA and cell line as the linear combination of gene-knockout and copy-number effects with coefficients giving the guide activities. Copy-number effects are fit with a linear piece-wise model in each cell line. Once all parameters have been fit, the inferred gene scores and guide activity scores are extracted and reported.

The CERES gene dependency data was further scaled to the -1 value of the median of common essential genes in each cell line, and then transformed into z-scores so each gene had mean 0 and variance 1. Next the first five principal components of the resulting data were removed, the prior means of genes were restored, and the data were scaled again so the median of common essentials in each cell lines was -1.

The pan-dependent genes were identified as those genes for whom 90% of cell lines rank the gene above a dependency cutoff determined from the central minimum in a histogram of gene ranks in their 90th percentile least dependent line. For each CERES gene score, the probability that the score represents a true dependency or not was inferred based on the expectation-maximization algorithm.

The differential dependency gene level scores for the Mito high vs. Mito low cell lines were quantified based on the Empirical Bayes (eBayes) statistics available from the limma package (Phipson et al., 2016)(Bioconductor v3.12 repository) with the significance cutoffs: abs(size effect) $\geq$ 0.3, P-value $\leq$ 0.05, adjusted P-value $\leq$ 0.10.

*Gene Set Enrichment Analysis (GSEA) for AML Mito dependencies*

The GSEA v4.1.0 software (Subramanian et al., 2005) was utilized to identify the Gene Ontology gene sets that have a significant overlap with the genes showing a differential dependency for the Mito-high vs. Mito-low AML cell lines in the CRISPR (Avana) 21Q2 dependency data. GSEA was performed across the collection of more than 7,300 GO BP gene sets available in the MSigDB v7.2 (Liberzon et al., 2011, 2015) database.

*Quantification and Statistical Analysis*

Clustering and proteomic cluster signature

Clustering of patients based on the proteome was performed by computing the pairwise Euclidean distance between patients. To avoid biases due to imputation strategies, we performed a pairwise deletion of missing observations when computing dissimilarities. Proteomic patient subgroups were identified by hierarchically clustering patients using complete linkage. Clusters were then characterized by performing a comparative pathway enrichment based on significantly upregulated proteins. To this end, we computed differentially regulated proteins between each cluster and the remaining patients using an empirical Bayesian approach, regularizing the protein-wise residual variances towards a common value (Phipson et al., 2016; Ritchie et al., 2015; Smyth Walter and Hall, 2009). This differential expression analysis was implemented using the eBayes method in the limma R package (version 3.44.3) and we filtered for upregulated proteins (i.e. proteins with a positive fold change) at an FDR of 5%. We then jointly characterized the individual clusters in a comparative analysis by profiling the top five significant terms (FDR<5%) enriched in the set of differentially upregulated proteins (Yu et al., 2012). To generate a more in-depth profiling of each cluster, we also performed a combined gene set enrichment analysis (GSEA) and gene set variation analysis (GSVA). First, a GSEA was performed based on the empirical Bayes moderated t-statistics computed with eBayes and for each cluster the top five significantly upregulated and top five significantly down-regulated terms (FDR<0.05) were retrieved. After GSEA analysis, we performed a post-hoc pathway redundancy analysis and first ranked enriched pathways by effect size. We then iteratively assessed whether a pathway had significant overlap with a pathway that had a larger effect size (i.e. whether it was redundant; significance via Fisher Test, FDR correction for multiple testing); we removed redundant pathways if normalized enrichment scores between the redundant pathway and its superseding pathway were consistent and broadcast superseding pathways down the ranked list, again ensuring consistency in normalized enrichment scores.

This set of significantly enriched non-redundant pathways was then used to generate a pathway-by-patient representation of the data in form of gene set variation scores using GSVA. To derive a proteomic cluster signature, we computed the up- and down-regulation of significantly variable pathways (FDR<5%, Kruskall-Wallis test). This up- and down-regulation was computed in from of a mean shift relative to the entire cohort using multiple regression analysis with GSVA scores as responses and one-hot-encoded cluster memberships as regressor.

All pathway analyses were based on performed based on Gene Ontology annotations (C5, retrieved using msigdbr). Heatmaps were generated using the "ComplexHeatmap" package v2.4.3. (Gu et al., 2016).

Multi-omics analysis and PCA

We first assessed associations between transcriptome and proteome via a correlation analysis. To this end, we computed Spearman rank correlation across patients for 5,702 gene-protein that were profiled in the proteome as well as the transcriptome. P-values were adjusted for multiple hypothesis testing using Benjamini-Hochberg and visualized with correlation coefficients in a volcano plot. To identify pathways enriched in gene-protein pairs with a high correlation, a GSEA was performed on the absolute correlation coefficient.

We ran a multi-omics factor analysis (MOFA) to jointly decompose variation in the proteome, transcriptome, cytogenetic data as well as mutation data. We refer to each data-layer as a view. MOFA is a group-factor-analysis-based statistical model that generates a joint representation of all data views via a weighted sum of latent factors. Latent factors are shared across data layers and each latent factor has a data-layer-specific set of weights. Let Y1,..,YM be data matrices of dimensions N × Dm representing M data views and where N is the number of patients and Dm the number of features (e.g proteins or genes) in data view m. MOFA then decomposes these matrices as a weighted sum:

$$Y^m = ZW^{mT} + \varepsilon^m \qquad m=1,...,M. \qquad (1)$$

Z represents the factor matrix, shared among all data views and $\mathbf{W}^m$ denote the data-view specific weight matrices. $\pounds^m$ is the view-specific residual noise term, with its form depending on the data-type. Factors, weights and noise parameters are inferred using approximate Bayesian inference. (Argelaguet et al., 2018)

Since MOFA is able to account for missing values in the modelling process, we used the unimputed protein expression data as model input for the proteome view. The mRNA view consisted of the log-transfromed, DESeq2 size-factor normalized expression of the 10,000 most variable genes (sorted by variance). The mutation view contained a total of 45 mutations (Fig. S4) and the cytogenetic view a total of 7 features, namely RUNX1-RUNX1T1, inv(16), CBF, KMT2A translocation, BCR/ABL, del(5) and inv(3). We performed a one-hot-encoding of mutations and cytogenetic features and modelled both views with a Bernoulli noise model. Proteome view and mRNA view were modelled using a Gaussian noise model. More specifically, for the Gaussian noise model, MOFA assumes i.i.d. heteroscedastic residuals for the m-th data view and d-th feature, i.e. $\varepsilon_d^m \sim N(0,1/\tau_d^m)$ with Gamma prior on the precision parameters $\tau_d^m$ .

MOFA models binary data using a Bernoulli likelihood for binary observations $y_{nd}^m$ for the n-th patient, d-th feature and m-th view. We fitted 10 MOFA models using the R package MOFA v1.6.1 with different initializations until convergence and selected the model with the best evidence lower bound (measuring model fit). For all hyperparameters, standard values as implemented in the MOFA package were used.

For downstream analysis we directly inspected factors (via beeswarm plots) and factor weights. In addition, we performed pathway analyses using a using a two-stage competitive test based on factor weights as implemented in the R package pcgse v0.1. (Frost et al., 2015) We visualized factor-specific pathways by computing patient-wise normalized enrichment scores via a weighted sum of genes annotated to a pathway.

We assessed associations between genetic classes and all proteomic classes using an exact Fisher test for all mutations and cytogenetic features described above. In addition, we quantified association to the chromatin/splicing class identified by Papaemmanuil et al. as well as to ELN risk(Papaemmanuil et al., 2016). We corrected for multiple testing using the Benjamini & Hochberg method and performed post-hoc Fisher tests for significant overall associations at an FDR of 5%.

We performed PCA on the unimputed proteome using the R package pcaMethods 1.82.0 via the ppca function; for RNA-seq data we used the prcomp function (R package stats 4.0.4). We performed pathway analyses of the loadings using a two-stage competitive test based on PC loadings as implemented in the R package pcgse v0.1 (Frost et al., 2015).

Survival analysis

Overall survival was measured from the date of diagnosis to the date of last follow-up or death. Kaplan-Meier univariate survival analysis was performed using the "survival" package v3.1-12 with R v4.0.2 as previously described (Terry M. Therneau and Patricia M. Grambsch, 2000). For the Cox proportional hazard regression model, the same package was used after testing for the proportional hazard assumption. The "survminer" v0.4.8 package was used for visualization of the Kaplan-Meier and Cox proportional hazard models.

Transcription factor plot

A list of human transcription factors with DBD annotation was retrieved from http://humantfs.ccbr.utoronto.ca/download.php (Lambert et al., 2018).

Kinases plot

A list of human kinases was retrieved from the human kinome project at https://kinase.com/human/kinome (Manning et al., 2002).

MitoCarta 3.0 heatmap

Version 3.0 of the human MitoCarta was retrieved from https://www.broadinstitute.org/mitocarta/mitocarta30-inventory-mammalian-mitochondrial-proteins-and-pathways . Gene-set variation analysis was used to transform the p-protein by n-sample log2 intensity matrix into a g-geneset by n-sample pathway enrichment matrix. Method = "gsva" with a gaussian kernel was used.

Machine learning-based Patient classification

We derived a predictive model classifying patients into Mito or Non-Mito using supervised machine learning. To this end, a binary classifier was trained based on the proteome of the discovery cohort. In order to generate a classifier that is robust to technical biases in the generation of proteomic expression data and can generalize across patient cohorts, we normalized the protein expression data of the discovery cohort using a global min-max normalization. We trained an ensemble of gradient boosted decision trees (xgboost v1.2.0) as interpretable classifier with high predictive power. In order to derive a small proteomic signature, we performed a nested 10-fold cross-validation with recursive feature selection in steps of 10 features, optimizing the f1-score of the inner folds of the nested cross-validation. The final signature consisted of 31 proteins that were selected in at least 3 out of 10 outer folds. We applied the classifier to the validation cohort, where 27 of the 31 proteins were quantified. Min-max normalization was performed based on the proteins in the signature only and classifier performance was evaluated using receiver-operator characteristics and precision-recall analysis.

To explain predictions, we computed the contribution of each protein in the signature towards a classification by following the decision paths in all trees of the ensemble and computing the output scores in the leaves of each tree as well as expected scores of the leaves' parent nodes; the contribution of a protein was then quantified as how much the expected score changes from parent to child using the eli5 v0.10.1 python package.

**Example 2**

Results

*Discovery of proteomic AML subtypes and their biological features*

Our multi-platform approach aiming at the identification of proteomic and proteogenomic AML subgroups included (i) protein expression, (ii) gene expression, as well as (iii) mutation and (iv) cytogenetic profiling. We performed in-depth proteome quantification of AML samples using our sensitive high-throughput single-run workflow (Kulak et al., 2014). In this study, we identified 9564 distinct protein groups in AML cells at a peptide and protein false discovery rate (FDR) of less than 1%. This covered a dynamic range of a protein signal spanning more than six orders of magnitude. Two cohorts of treatment-naïve patients were profiled: A discovery cohort of 177 patients (proteome coverage 6522 proteins) and an independent validation cohort of 75 patients (proteome coverage 6754 proteins). In the discovery cohort, protein expression was analyzed quantitatively by label-free mass-spectrometry in Ficoll-enriched bone-marrow blasts from 177 untreated patients with AML who were all treated by intensive induction chemotherapy (cytarabine and daunorubicin; 7+3 regimen). This cohort included de novo as well as secondary and therapy-related AML; it covered all European leukaemia net (ELN) 2017 cytogenetic risk categories and showed outcomes similar to those of previously published representative AML cohorts (Table 1 and Figure 1A). In addition, targeted sequencing of somatic mutations revealed mutation frequencies similar to those previously reported in AML. Cytogenetic aberrations were determined by conventional metaphase karyotyping and confirmed by fluorescence in situ hybridization during routine diagnostics.

To characterize patient subpopulations within the proteomic landscape in the discovery cohort, we performed a distance-based analysis of the quantitative proteome data. We computed pairwise distances between patients and performed unsupervised hierarchical clustering to identify coherent proteomic subgroups of similar patients (Figure 1B). This data-driven approach revealed six distinct proteomic subgroups (Clusters 1-6, C1-6). Although we found clearly separated proteomic clusters, none of them showed an exclusive association with any specific mutation-type or cytogenetic aberration. Next, we performed a pathway-based characterization of all proteomic clusters, to investigate specific biological features of individual proteomic subgroups. We identified biological profiles for all six clusters. A comparative cluster analysis revealed that clusters 1 and 2 had a common profile dominated by highly expressed mitochondrial proteins and mitochondria-related processes, distinct from all other clusters. Therefore, we merged patients from those clusters to form a larger subpopulation (frequency 14%). We confirmed the unique mitochondrial profile of this merged cluster by performing a gene-set variation analysis (GSVA) of all patients and analysing the cluster-wise enrichment profiles of pathways that were differentially regulated between the 5 consolidated clusters (false discovery rate (FDR) < 0.01; Figure 1C); we refer to the merged cluster as the Mito-cluster (C-Mito). Notably, enrichment of mitochondrial proteins was not correlated with mitochondrial mass or higher proliferation rates of the respective AML blasts as revealed by an assessment of Ki-67 expression by expert pathologists who were blinded for the proteomic cluster assignment; therefore C-Mito does not just reflect more proliferative disease. Also, C-Mito was not enriched for any specific FAB subtype, indicating that C-Mito is not linked to particular cellular differentiation stages. Clusters 3-6 are associated with processes related to RNA-processing (C3), and myeloid leukocyte activation and immune response (C4-6). Similar to C-Mito, C4 and C6 were also not linked to any FAB categories, while C3 was enriched for undifferentiated (M0) and minimally differentiated (M1) AML and C5 for myelomonocytic (M4) AML, respectively.

Since AML cells originate from hematopoietic stem and progenitor cells, we have next profiled protein expression in CD34-positive bone marrow cells from 13 healthy donors to investigate whether certain proteomic features are shared between both cell types. We found that the pathways enriched in the loadings of PC1, which explains most variance in each cell type, are markedly different between the CD34-positive cells and the AML cells. In contrast PC2 revealed commonalities between their proteomes reflected by terms related to neutrophil function and RNA metabolism, indicating that some proteomic features of AML cells are shared with CD34-positive bone marrow cells.

Our resource data-set allows assessment of protein expression patterns in proteomic and genomic AML subtypes. To obtain more detailed insight into proteomic differences among the proteomic AML subtypes identified, we explored the expression patterns of transcription factors and kinases. We found significant differences in expression of these protein classes among the proteomic subtypes (Figure 1D,E). For example, transcription factors of the signal transducers and activators of transcription (STAT) family were less strongly expressed in C-Mito (Figure 1D), which is interesting in the light of previous studies showing that STATs are involved in the regulation of mitochondrial function (Meier and Larner, 2014). Similarly, differences in the expression of kinases were observed. For example, a significant upregulation of pyruvate dehydrogenase kinases (PDHK) in C-Mito, among others, pointed toward a specific metabolic wiring of this AML subtype (Figure IE).

To gain an integrated proteogenomic perspective, we next performed RNA-sequencing to characterize (in addition to mutations, cytogenetics and proteome) the transcriptome of the AML blasts from our discovery cohort (Table 1). We obtained transcriptome data for 136 patients in the discovery cohort, and 60,523 unique transcripts were quantified.

The transcriptomic data obtained were strongly correlated with previously published gene expression analyses in AML. We first compared protein- and RNA-expression data in our cohort. We found a significant correlation (FDR<1%) for 1,588 out of 5,702 gene-protein pairs that were measured in both the transcriptome and the proteome, confirming a large heterogeneity across genes in terms of the correlation between RNA and protein in AML blasts. Highly correlated gene-protein pairs were enriched for pathways related to immune response and microbial defense, while for example genes associated with the mitochondrion had a significantly lower absolute correlation than other genes did (p=0.005, Wilcoxon rank sum test).

To investigate whether the cluster-specific proteomic pathway profiles were reflected in the transcriptome, we repeated a GSVA for the transcriptomic data and quantified the intra-cluster correlation of enrichment scores between proteome and transcriptome. For most pathways and clusters - except for a few processes such as those related to antibacterial and antimicrobial defense mechanisms - proteomic enrichment scores were not significantly correlated to transcriptomic enrichment scores (Figure 1C) highlighting that RNA and protein expression seem to be largely uncoupled in AML.

*Multi-Omics integration reveals the proteogenomic landscape of AML*

To disentangle inter-patient variability more comprehensively and to understand the relationships between the different molecular layers, we next performed an integrated multi-omics factor analysis (MOFA). This analysis determined coordinated biological processes that were driving variation in several data layers (i.e. protein and gene expression, mutations, cytogenetics). Moreover, it also identified biological processes that were only reflected in a single data layer. In brief, MOFA jointly decomposes all data layers into distinct sources of variation in an interpretable linear model. These sources of variation are represented by latent factors (LFs) in the sense of generalized principal components. We referred to LFs that explain variance in at least one data layer as being active in that layer, and we ordered LFs by overall variance explained akin to principal components. MOFA identified a total of 28 LFs driving variation between patients (Figure 2A), with LF1 explaining most variance and LF28 explaining least variance across all data layers. While most LFs explained variance in several data layers in a coordinated fashion, MOFA also identified a set of 17 unique factors that were active in only a single data layer, namely 12 in the proteome and 5 in the transcriptome.

The most dominant factor (LF1) was active in all data layers and represents the well-characterized dimension of AML heterogeneity related to NPM1 mutations and HOX gene signatures, in turn indicating that NPM1 mutations and strongly expressed Hox-dependent genes co-occur in a significant number of AML cases. NPM1 mutations have previously been reported to dysregulate the Hox-gene transcription network, and MOFA here linked the identified NPM1 mutations to the Hox gene transcriptome signature (Figure 2B,C) (Armstrong et al., 2002; Bernt and Armstrong, 2011; Huang et al., 2012). We therefore refer to this factor as LF1-NPM1/HOX. Notably, LF1-NPM1/HOX also detected coordinated variation related to the major histocompatibility complex in the proteome as well as the transcriptome. An HLA class I bias has recently been described for NPM1-mutant AML and our data suggest that there may be a molecular connection between NMP1 mutations and specific HLA-related RNA and protein profiles (Kuzelová et al., 2018) (Figure 2B). Importantly, MOFA identified positive associations between different data layers, and also inverse couplings. For example, LF1-NPM1/HOX revealed a mutually exclusive pattern for NPM1 mutations and cytogenetic characteristics of core binding factor (CBF) leukemia (t(8;21) and inv(16)). This confirms previous findings that NPM1 mutations occur almost exclusively in AML with normal karyotype and are rarely found in patients with reciprocal translocations, especially the CBF-AML-defining aberrations inv(16) and t(8;21) (Thiede et al., 2006). MOFA revealed this link through a high weight of these CBF features in the cytogenetic data view.

Similarly, LF4-RUNX1-RUNX1T1 and LF12-TET2 uncovered coordinated variation driven by common genomic aberrations that are also reflected in the transcriptome and the proteome. Inspection of the content of LF4 revealed that the RUNX1-RUNX1T1 translocation was driving cytogenetic variation. While RUNX1 was the gene with the highest weight in the transcriptome, variation in the proteome was driven by CEBP`@` and MAFG, both of which have been linked to the RUNX1-RUNX1T1-positive AML (Pabst and Mueller, 2009; Ptasinska et al., 2019). Content for LF12-TET2 linked TET2 in the mutation data layer to Caspase 3 in the proteome and PRDM16 in the transcriptome data layers, respectively, again revealing a known association (Guo et al., 2019; Ko et al., 2013).

We next used MOFA to focus on the inter-patient variability reflected in the proteome only. Here the most dominant factor was LF6; its content revealed that genes associated with the mitochondrion drive variation in LF6-mito (Figure 2D). Remarkably, the intrinsic order of patients along LF6-mito showed that LF6-mito largely detected patients assigned to C-Mito (Figure 2E). We further confirmed the mitochondrial signature of LF6 by quantifying the association between LF6-mito and a mitochondrial principal component (PC1-Mito) that was computed on the basis of proteins annotated to the mitochondrion, revealing a significant correlation (rho=0.88, Figure 2F). In contrast, no latent factor that was active in a data layer other than the proteome was able to identify those C-Mito patients. To provide further confirmation that patients with a proteomic C-Mito signature could not be detected through the transcriptome, we performed independent principal component analyses (PCA) based on genes annotated to the mitochondrion for the proteome and the transcriptome (Figure 2G). C-Mito patients were again only detected by the proteome data. Similarly, the proteomic mitochondrial signature could not be detected in the transcriptome of CD34-positive and negative leukemic stem cells which were purified from Mito-AML bulk samples from our cohort. This illustrates that post-transcriptional regulation can lead to a substantial uncoupling of transcript and protein expression levels.

Taken together, these results show that the subgroup of patients assigned to C-Mito is reflected in the proteome only, highlighting the importance of proteomics as discovery tool. Similarly, LF7 and LF8 largely recover the clusters identified in the distance-based analysis of the proteome, as LF7 and LF8 identify patients in C3 and C4, respectively. Individual weights for all 28 factors are presented in the supplement (Table S5).

*Clinical and genomic attributes of the proteomic AML subtypes*

After having identified five proteomic AML subtypes and their specific proteogenomic features, we explored their clinical relevance. Clinical correlation analyses revealed that two of the five proteomic AML subtypes identified have prognostic relevance. Patients belonging to C-Mito showed shorter overall survival (OS, Figure 3A; p=0.0034). Importantly, both C-Mito subclusters (shown in Figure 1) have an independent and significant survival phenotype. In a multivariate analysis that included patient age and the cytogenetic ELN2017 risk categories, the distinction regarding the Mito subgroup contributed independently, as a poor risk factor, to overall survival (hazard ratio 3.6; 95% CI, 1.92 to 6.62; p<0.001; Figure 3B). Basic patient characteristics were similar in the Mito and non-Mito groups, as no significant difference between the two groups was observed for age, sex distribution or the rate of allogeneic stem-cell transplantation (Table S3. In contrast, differences were observed regarding cytogenetic risk groups and mutation profiles (Figure 3C). Surprisingly, although characterized by shorter OS, C-Mito was enriched for favorable risk disease characteristics according to the ELN2017 risk classification, while non-Mito AML showed an enrichment of adverse risk features, including an enrichment of secondary AML (Figure 3C). Genetically, we observed a trend toward a higher frequency of NPM1 mutations in the Mito-group (12/25, 48%) as compared with the non-Mito group (44/152, 29.9%; Figure 3C). Clinically, the complete remission rate (CR1) was significantly lower in the Mito than in the non-Mito group (Mito: 13/25, 52%; non-Mito: 112/152, 73.7%; P<0.05), while relapse-free survival did not differ between the two groups indicating an immediate chemotherapy resistance phenotype in the Mito group.

In contrast to C-Mito, OS was longer in C5 than in non-C5 patients (Figure 3D; p=0.011) and the multivariate analysis identified C5 as an independent favorable risk factor (hazard ratio 0.45; 95% CI, 0.26 to 0.79; p=0.006; Figure 3E). Basic patient characteristics, including age, sex distribution and the rate of allogenic transplantation as well as the cytogenetic risk groups, were balanced between these groups. Genetically, C5 showed an enhanced frequency of DNMT3A mutations, which might affect the proteome and disease outcome of the patients in the C5 group (Figure 3F). Overall, C5 was identified as the largest proteomic cluster and it lacked, in contrast to C-Mito, a unique protein expression signature pointing toward a certain degree of heterogeneity within this cluster. To resolve this heterogeneity, we performed a more in-depth bioinformatic characterization of C5 and explored biological features of this favorable risk patient subset. We further subclustered C5 in order to detect more biologically specific groups within this large cluster. This yielded three C5 subclusters with two of them, subclusters C5.1 and C5.3, having more favorable survival outcomes. A multivariate survival analysis revealed that the survival phenotype of subcluster C5.1 is confounded by ELN cytogenetic risk categories (hazard ratio 0.53; 95% CI, 0.22 to 1.2; p=0.143,). In contrast, the multivariate analysis identified C5.3 as an independent favorable risk factor (hazard ratio 0.23, 95% CI, 0.055 to 0.93; p = 0.04). A gene-set enrichment analysis revealed that C5.3 was characterized by a negative regulation of NOTCH4 signaling. Interestingly, high levels of NOTCH4 expression were found to be an independent prognostic factor for poorer survival in AML patients (Kamga et al., 2019). Hence negative regulation of NOTCH4 signaling could in parts explain the favorable prognosis of C5.3 patients. The remaining subcluster C5.2 showed no survival phenotype as did clusters C3, C4 and C6.

*Validation of the Mito-AML subtype in an independent patient cohort*

Since our discovery analysis identified proteomic subtypes with clinical relevance, we next validated our results in an independent, genetically characterized cohort of 75 AML patients who, as in the discovery cohort, were uniformly treated by intensive induction therapy (Table 1). Because of its smaller size this cohort was restricted to patients with core binding factor leukemia and patients with NPM1 mutations (with and without FLT3-ITD) (Table 1). For quantitative analysis of the proteome of Ficoll-enriched pretherapeutic bone-marrow blasts an alternative, Super-SILAC-based, quantitative mass-spectrometric workflow was used. This method has very high quantification accuracy and hence we used this alternative quantitative mass-spectrometry approach, to achieve a robust validation, particularly by ruling out any technology-related artefacts potentially related to our label-free proteomic approach (Bohnenberger et al., 2018; Geiger et al., 2010).

In the validation cohort, a distance-based hierarchical clustering analysis of the quantitative proteome data revealed three independent clusters (Figure 4A). A comparative cluster enrichment analysis validated C-Mito (frequency 20%) and an enrichment of NPM1 mutations in this cluster (Figure 4A,B). To confirm that the C-Mito cluster in the validation cohort indeed corresponds to the C-Mito cluster in the discovery cohort, we performed a machine-learning-based analysis directly linking the two patient cohorts. To this end, we trained a predictive model to classify C-Mito patients based on the proteome of the discovery cohort only, using a recursive feature selection approach. We then applied the trained model to the validation cohort, prospectively classifying all patients into 'C-Mito' and 'non-Mito' on the basis of a set of 27 proteins. Remarkably, 14 out of 15 patients in the validation cohort who had been identified as C-Mito by unsupervised clustering and pathway analysis were classified by our prediction model as C-Mito (recall of 93.3% with a precision of 100%; Figure 4C). To elucidate further the predictive cross-cohort Mito-signature driving the Mito-classifier, we computed the contribution of each protein in the signature to the classifications of all patients in the validation cohort. Of the 5 proteins with the largest contributions to C-Mito prediction, 2 proteins were related to the mitochondrion (CYFIB-related Rac1 interactor B, CYRIB) and mitochondrial 39S ribosomal protein L19 (MRPL19) and two to a nuclear pore complex (nuclear pore complex protein 88 (NUP88), nucleoprotein TPR), representing a condensed predictive signature of C-Mito and a first step toward establishing a diagnostic marker panel (Figure 4D).

We further used the validation cohort to confirm the poor outcome of C-Mito patients when treated with intensive induction therapy. As for the discovery cohort, we found that overall survival was significantly shorter for C-Mito patients compared with non-Mito patients, both for predicted C-Mito patients (Figure 4E) and for those identified as C-Mito by unsupervised clustering (Figure 4F,G).

*The Mito-AML-defining proteome network*

To gain further insight into the biological characteristics of C-Mito, we extracted the top 100 proteins that showed a significant expression difference when comparing C-Mito with all other proteomic clusters. The majority of these proteins belonged to one of three biological categories, namely mitochondria, the nuclear envelope and chromatin regulators (Figure 5A). The strong interconnectivity of these proteins was revealed by superimposing previously published protein interactions to obtain a protein interaction network for the proteins that characterize C-Mito. With regard to mitochondria, C-Mito was characterized by high expression levels of almost all mitochondrial proteins including the respiratory complexes I-VI, and in accordance with our MOFA analysis this phenomenon was only observed at the level of protein, and not of RNA (Figure 5B-E). Thus, our data point clearly toward a post-transcriptional mechanism leading to the accumulation of mitochondrial proteins in C-Mito. Similarly, nuclear pore components were upregulated exclusively at the protein level.

*Mitochondrial complex-I dependence of Mito-AML*

Given that our proteomic approach has identified Mito-AML as a high-risk AML subtype, we investigated whether this subtype has specific pathomechanistic features. To this end, we first profiled protein expression in 25 leukemia cell lines to investigate whether proteomic profiling can distinguish Mito from non-Mito AML cell lines and we could use them as models for functional research. In these cell lines, protein expression was quantified by label-free mass-spectrometry leading to the identification of 8573 proteins. Unsupervised clustering identified 9 AML cell lines as Mito-high and 9 as Mito-low (Figure 6A). Because differential expression of mitochondrial proteins was also observed in AML cell lines, we used them as models to investigate whether the proteomic Mito-feature has a functional relevance.

We first integrated functional genomic data from publicly available CRISPR/Cas-9 loss-of-function screens to elucidate the vulnerabilities in Mito-high versus Mito-low cell lines. For 10 AML lines of the DepMap CRISPR dataset we had proteomic data available, and of those 6 fell into the Mito and 4 into the non-Mito categories, respectively. The CRISPR screens showed that the dependency on mitochondrial complex I genes was much stronger in the Mito versus non-Mito lines (Figure 6B), while genes belonging to complexes II-V showed no difference (Figure 6C). In addition to complex I, genes involved in DNA repair, histone acetylation and mTOR signaling pathways were stronger dependencies in the Mito cell lines (Figure 6B, S6D).

We therefore profiled the in vitro sensitivity of Mito-high and -low cell lines to the complex I inhibitor mubritinib, as well as to venetoclax and to combined venetoclax/azacytidine treatment, all of which also target mitochondrial respiration. This revealed a significant hypersensitivity of Mito-high AML lines to these drugs (Figure 6D, S6F). Moreover, we conducted a Seahorse metabolic analysis and found that Mito-high AML lines showed a higher spare respiratory capacity and stronger complex I dependent respiration (Figure 6E). Stronger complex I-dependent respiration was furthermore validated in primary samples from our discovery cohort (Figure 6E). These data confirm stronger complex I dependent respiration in Mito AML cells compared with non-Mito cells. In line with these results, venetoclax treatment led to a stronger reduction in the oxygen consumption rate in Mito-compared with non-Mito cells (Figure 6G). Together, these results indicate that Mito-AML cells have a specific wiring of their mitochondrial respiration which makes them more vulnerable to drugs that target complex I.

**Further References**

Argelaguet, R., Velten, B., Arnol, D., Dietrich, S., Zenz, T., Marioni, J.C., Buettner, F., Huber, W., and Stegle, O. (2018). Multi-Omics Factor Analysis-a framework for unsupervised integration of multi-omics data sets. Mol. Syst. Biol. 14.

Armstrong, S.A., Staunton, J.E., Silverman, L.B., Pieters, R., Den Boer, M.L., Minden, M.D., Sallan, S.E., Lander, E.S., Golub, T.R., and Korsmeyer, S.J. (2002). MLL translocations specify a distinct gene expression profile that distinguishes a unique leukemia. Nat. Genet. 30, 41-47.

Bernt, K.M., and Armstrong, S.A. (2011). Targeting epigenetic programs in MLL-rearranged leukemias. Hematology Am. Soc. Hematol. Educ. Program 2011, 354-360.

Bhatt, S., Pioso, M.S., Olesinski, E.A., Yilma, B., Ryan, J.A., Mashaka, T., Leutz, B., Adamia, S., Zhu, H., Kuang, Y., et al. (2020). Reduced Mitochondrial Apoptotic Priming Drives Resistance to BH3 Mimetics in Acute Myeloid Leukemia. Cancer Cell.

Blomen, V.A., Májek, P., Jae, L.T., Bigenzahn, J.W., Nieuwenhuis, J., Staring, J., Sacco, R., Van Diemen, F.R., Olk, N., Stukalov, A., et al. (2015). Gene essentiality and synthetic lethality in haploid human cells. Science (80-. ). 350, 1092-1096.

Bohnenberger, H., Kaderali, L., Strobel, P., Yepes, D., Plessmann, U., Dharia, N. V, Yao, S., Heydt, C., Merkelbach-Bruse, S., Emmert, A., et al. (2018). Comparative proteomics reveals a diagnostic signature for pulmonary head-and-neck cancer metastasis. EMBO Mol. Med. 10.

Capes-Davis, A., Reid, Y.A., Kline, M.C., Storts, D.R., Strauss, E., Dirks, W.G., Drexler, H.G., MacLeod, R.A.F., Sykes, G., Kohara, A., et al. (2013). Match criteria for human cell line authentication: Where do we draw the line? Int. J. Cancer 132, 2510-2519.

Chao, M.P., Takimoto, C.H., Feng, D.D., McKenna, K., Gip, P., Liu, J., Volkmer, J.P., Weissman, I.L., and Majeti, R. (2020). Therapeutic Targeting of the Macrophage Immune Checkpoint CD47 in Myeloid Malignancies. Front. Oncol. 9.

Chopra, S., Ramkissoon, K., and Anderson, D.C. (2013). A systematic quantitative proteomic examination of multidrug resistance in Acinetobacter baumannii. J. Proteomics 84, 17-39.

Coscia, F., Lengyel, E., Duraiswamy, J., Ashcroft, B., Bassani-Sternberg, M., Wierer, M., Johnson, A., Wroblewski, K., Montag, A., Yamada, S.D., et al. (2018). Multi-level Proteomics Identifies CT45 as a Chemosensitivity Mediator and Immunotherapy Target in Ovarian Cancer. Cell 175, 159-170.e16.

Cox, J., Hein, M.Y., Luber, C.A., Paron, I., Nagaraj, N., and Mann, M. (2014). Accurate proteomewide label-free quantification by delayed normalization and maximal peptide ratio extraction, termed MaxLFQ. Mol. Cell. Proteomics 13, 2513-2526.

Dempster, J.M., Rossen, J., Kazachkova, M., Pan, J., Kugener, G., Root, D.E., and Tsherniak, A. (2019). Extracting Biological Insights from the Project Achilles Genome-Scale CRISPR Screens in Cancer Cell Lines. BioRxiv 720243.

DiNardo, C.D., Stein, E.M., de Botton, S., Roboz, G.J., Altman, J.K., Mims, A.S., Swords, R., Collins, R.H., Mannis, G.N., Pollyea, D.A., et al. (2018). Durable Remissions with Ivosidenib in IDH1-Mutated Relapsed or Refractory AML. N. Engl. J. Med. NEJMoa1716984.

DiNardo, C.D., Jonas, B.A., Pullarkat, V., Thirman, M.J., Garcia, J.S., Wei, A.H., Konopleva, M., Döhner, H., Letai, A., Fenaux, P., et al. (2020). Azacitidine and Venetoclax in Previously Untreated Acute Myeloid Leukemia. N. Engl. J. Med. 383, 617-629.

Doench, J.G., Fusi, N., Sullender, M., Hegde, M., Vaimberg, E.W., Donovan, K.F., Smith, I., Tothova, Z., Wilen, C.,

Orchard, R., et al. (2016). Optimized sgRNA design to maximize activity and minimize off-target effects of CRISPR-Cas9. Nat. Biotechnol. 34, 184-191.

Döhner, H., Estey, E., Grimwade, D., Amadori, S., Appelbaum, F.R., Büchner, T., Dombret, H., Ebert, B.L., Fenaux, P., Larson, R.A., et al. (2017). Diagnosis and management of AML in adults: 2017 ELN recommendations from an international expert panel. Blood 129, 424-447.

Farge, T., Saland, E., de Toni, F., Aroua, N., Hosseini, M., Perry, R., Bosc, C., Sugita, M., Stuani, L., Fraisse, M., et al. (2017). Chemotherapy-Resistant Human Acute Myeloid Leukemia Cells Are Not Enriched for Leukemic Stem Cells but Require Oxidative Metabolism. Cancer Discov. 7, 716-735.

Frost, H.R., Li, Z., and Moore, J.H. (2015). Principal component gene set enrichment (PCGSE). BioData Min. 8, 25.

Geiger, T., Cox, J., Ostasiewicz, P., Wisniewski, J.R., and Mann, M. (2010). Super-SILAC mix for quantitative proteomics of human tumor tissue. Nat. Methods 7, 383-385.

Guo, X., Zhong, W., Chen, Y., Zhang, W., Ren, J., and Gao, A. (2019). Benzene metabolites trigger pyroptosis and contribute to haematotoxicity via TET2 directly regulating the Aim2/Casp1 pathway. EBioMedicine 47, 578-589.

Hart, T., Brown, K.R., Sircoulomb, F., Rottapel, R., and Moffat, J. (2014). Measuring error rates in genomic perturbation screens: gold standards for human functional genomics. Mol. Syst. Biol. 10, 733.

Hart, T., Chandrashekhar, M., Aregger, M., Steinhart, Z., Brown, K.R., MacLeod, G., Mis, M., Zimmermann, M., Fradet-Turcotte, A., Sun, S., et al. (2015). High-Resolution CRISPR Screens Reveal Fitness Genes and Genotype-Specific Cancer Liabilities. Cell 163, 1515-1526.

Hartmann, S., Agostinelli, C., Klapper, W., Korkolopoulou, P., Koch, K., Marafioti, T., Piccaluga, P.P., Patsouris, E., Pileri, S., and Hansmann, M.L. (2011). Revising the historical collection of epithelioid cell-rich lymphomas of the Kiel Lymph Node Registry: What is Lennert's lymphoma nowadays? Histopathology 59, 1173-1182.

Huang, Y., Sitwala, K., Bronstein, J., Sanders, D., Dandekar, M., Collins, C., Robertson, G., MacDonald, J., Cezard, T., Bilenky, M., et al. (2012). Identification and characterization of Hoxa9 binding sites in hematopoietic cells. Blood 119, 388-398.

Huber, W., Carey, V.J., Gentleman, R., Anders, S., Carlson, M., Carvalho, B.S., Bravo, H.C., Davis, S., Gatto, L., Girke, T., et al. (2015). Orchestrating high-throughput genomic analysis with Bioconductor. Nat. Methods 12, 115-121.

Jayavelu, A.K., Schnöder, T.M., Perner, F., Herzog, C., Meiler, A., Krishnamoorthy, G., Huber, N., Mohr, J., Edelmann-Stephan, B., Austin, R., et al. (2020). Splicing factor YBX1 mediates persistence of JAK2-mutated neoplasms. Nature 588, 157-163.

Kamga, P.T., Collo, G.D., Resci, F., Bazzoni, R., Mercuri, A., Quaglia, F.M., Tanasi, I., Delfino, P., Visco, C., Bonifacio, M., et al. (2019). Notch Signaling Molecules as Prognostic Biomarkers for Acute Myeloid Leukemia. Cancers 2019, Vol. 11, Page 1958 11, 1958.

Ko, M., An, J., Bandukwala, H.S., Chavez, L., Äijö, T., Pastor, W.A., Segal, M.F., Li, H., Koh, K.P., Lähdesmäki, H., et al. (2013). Modulation of TET2 expression and 5-methylcytosine oxidation by the CXXC domain protein IDAX. Nature 497, 122-126.

Kulak, N.A., Pichler, G., Paron, I., Nagaraj, N., and Mann, M. (2014). Minimal, encapsulated proteomic-sample processing applied to copy-number estimation in eukaryotic cells. Nat. Methods 11, 319-324.

Kuželová, K., Brodská, B., Schetelig, J., Rollig, C., Rácil, Z., Walz, J.S., Helbig, G., Fuchs, O., Vraná, M., Pecherková, P., et al. (2018). Association of HLA class I type with prevalence and outcome of patients with acute myeloid leukemia and mutated nucleophosmin. PLoS One 13.

Lachowiez, C.A., Loghavi, S., Kadia, T.M., Daver, N., Borthakur, G., Pemmaraju, N., Naqvi, K., Alvarado, Y., Yilmaz, M., Short, N., et al. (2020). Outcomes of older patients with NPM1-mutated AML: Current treatments and the promise of venetoclax-based regimens. Blood Adv. 4, 1311-1320.

Lambert, S.A., Jolma, A., Campitelli, L.F., Das, P.K., Yin, Y., Albu, M., Chen, X., Taipale, J., Hughes, T.R., and Weirauch, M.T. (2018). The Human Transcription Factors. Cell 172, 650-665.

Liberzon, A., Subramanian, A., Pinchback, R., Thorvaldsdóttir, H., Tamayo, P., and Mesirov, J.P. (2011). Molecular signatures database (MSigDB) 3.0. Bioinformatics 27, 1739-1740.

Liberzon, A., Birger, C., Thorvaldsdóttir, H., Ghandi, M., Mesirov, J.P., and Tamayo, P. (2015). The Molecular Signatures Database Hallmark Gene Set Collection. Cell Syst. 1, 417-425.

Love, M.I., Huber, W., and Anders, S. (2014). Moderated estimation of fold change and dispersion for RNA-seq data with DESeq2. Genome Biol. 15.

Manning, G., Whyte, D.B., Martinez, R., Hunter, T., and Sudarsanam, S. (2002). The protein kinase complement of the human genome. Science (80-. ). 298, 1912-1934.

McInnes, L., Healy, J., and Melville, J. (2018). UMAP: Uniform Manifold Approximation and Projection for Dimension Reduction.

Meier, J.A., and Larner, A.C. (2014). Toward a new STATe: The role of STATs in mitochondrial function. Semin. Immunol. 26, 20-28.

Meyers, R.M., Bryan, J.G., Mcfarland, J.M., Weir, B.A., Sizemore, A.E., Xu, H., Dharia, N. V, Montgomery, P.G., Cowley, G.S., Pantel, S., et al. (2017). Computational correction of copy number effect improves specificity of CRISPR - Cas9 essentiality screens in cancer cells. Nat. Publ. Gr.

Pabst, T., and Mueller, B.U. (2009). Complexity of CEBPA dysregulation in human acute myeloid leukemia. Clin. Cancer Res. 15, 5303-5307.

Paczulla, A.M., Rothfelder, K., Raffel, S., Konantz, M., Steinbacher, J., Wang, H., Tandler, C., Mbarga, M., Schaefer, T., Falcone, M., et al. (2019). Absence of NKG2D ligands defines leukaemia stem cells and mediates their immune evasion. Nature 572, 254-259.

Papaemmanuil, E., Gerstung, M., Bullinger, L., Gaidzik, V.I., Paschka, P., Roberts, N.D., Potter, N.E., Heuser, M., Thol, F., Bolli, N., et al. (2016). Genomic Classification and Prognosis in Acute Myeloid Leukemia. N. Engl. J. Med. 374, 2209-2221.

Pei, S., Minhajuddin, M., Adane, B., Khan, N., Stevens, B.M., Mack, S.C., Lai, S., Rich, J.N., Inguva, A., Shannon, K.M., et al. (2018). AMPK/FIS1-Mediated Mitophagy Is Required for Self-Renewal of Human AML Stem Cells. Cell Stem Cell 23, 86-100.e6.

Phipson, B., Lee, S., Majewski, I.J., Alexander, W.S., and Smyth, G.K. (2016). Robust hyperparameter estimation protects against hypervariable genes and improves power to detect differential expression. Ann. Appl. Stat. 10, 946-963.

Picelli, S., Faridani, O.R., Björklund, Å.K., Winberg, G., Sagasser, S., and Sandberg, R. (2014). Full-length RNA-seq from single cells using Smart-seq2. Nat. Protoc. 2013 91 9, 171-181.

PJ, V., RG, V., MA, B., CA, E., S, B. van W. van D.-K., JM, B., HB, B., MJ, M., PJ, van der S., B, L., et al. (2004). Prognostically useful gene-expression profiles in acute myeloid leukemia. N. Engl. J. Med. 350, 1617-1628.

Ptasinska, A., Pickin, A., Assi, S.A., Chin, P.S., Ames, L., Avellino, R., Groschel, S., Delwel, R., Cockerill, P.N., Osborne, C.S., et al. (2019). RUNX1-ETO Depletion in t(8;21) AML Leads to C/EBPα-and AP-1-Mediated Alterations in Enhancer-Promoter Interaction. Cell Rep. 28, 3022-3031.e7.

R Core Team (2020). R: A Language and Environment for Statistical Computing.

Rath, S., Sharma, R., Gupta, R., Ast, T., Chan, C., Durham, T.J., Goodman, R.P., Grabarek, Z., Haas, M.E., Hung, W.H.W., et al. (2021). MitoCarta3.0: An updated mitochondrial proteome now with sub-organelle localization and pathway annotations. Nucleic Acids Res. 49, D1541-D1547.

Ritz, C., Baty, F., Streibig, J.C., and Gerhard, D. (2015). Dose-Response Analysis Using R. PLoS One 10, e0146021.

Sanjana, N.E., Shalem, O., and Zhang, F. (2014). Improved vectors and genome-wide libraries for CRISPR screening. Nat. Methods 11, 783-784.

Shafer, D., and Grant, S. (2016). Update on rational targeted therapy in AML. Blood Rev. 30, 275-283.

Solh, M., Yohe, S., Weisdorf, D., and Ustun, C. (2014). Core-binding factor acute myeloid leukemia: Heterogeneity, monitoring, and therapy. Am. J. Hematol. 89, 1121-1131.

Stone, R.M., Mandrekar, S.J., Sanford, B.L., Laumann, K., Geyer, S., Bloomfield, C.D., Thiede, C., Prior, T.W., Döhner, K., Marcucci, G., et al. (2017). Midostaurin plus Chemotherapy for Acute Myeloid Leukemia with a FLT3 Mutation . N. Engl. J. Med. 377, 454-464.

Subramanian, A., Tamayo, P., Mootha, V.K., Mukherjee, S., Ebert, B.L., Gillette, M.A., Paulovich, A., Pomeroy, S.L., Golub, T.R., Lander, E.S., et al. (2005). Gene set enrichment analysis: A knowledge-based approach for interpreting genome-wide expression profiles. Proc. Natl. Acad. Sci. U. S. A. 102, 15545-15550.

Terry M. Therneau, and Patricia M. Grambsch (2000). Modeling Survival Data: Extending the {C}ox Model (New York: Springer).

Thiede, C., Koch, S., Creutzig, E., Steudel, C., Illmer, T., Schaich, M., and Ehninger, G. (2006). Prevalence and prognostic impact of NPM1 mutations in 1485 adult patients with acute myeloid leukemia (AML). Blood 107, 4011-4020.

Tyanova, S., Temu, T., and Cox, J. (2016a). The MaxQuant computational platform for mass spectrometry-based shotgun proteomics. Nat. Protoc. 11, 2301-2319.

Tyanova, S., Temu, T., Sinitcyn, P., Carlson, A., Hein, M.Y., Geiger, T., Mann, M., and Cox, J. (2016b). The Perseus computational platform for comprehensive analysis of (prote)omics data. Nat. Methods 13, 731-740.

THE FIRST-IN-CLASS ANTI-CD47 ANTIBODY MAGROLIMAB COMBINED WITH.... EHA Library. David S. Jun 12 2020; 295007.

**Claims**

1. A method of identifying sub-forms of acute myeloid leukemia (AML), said method comprising

    (a) determining protein expression levels of a plurality of proteins in a cohort of patients suffering from AML; and
    (b) classifying said patients according to said protein expression levels;

    thereby obtaining classes of patients which define sub-forms of AML.

2. The method of claim 1, wherein said plurality of proteins comprises or consists of proteins detectable by proteomics of AML cells including of a single AML cell, preferably mitochondrial proteins detectable by said proteomics.

3. The method of claim 1 or 2, wherein said sub-forms differ from each other with regard to at least one of overall survival, prognosis, response to chemotherapy, and treatment of choice.

4. Use of a set of proteins for determining prognosis of and/or treatment of choice for a patient suffering from AML or for selecting said patient for a specific therapy, wherein said set of proteins comprises or consists of

    (i) at least three mitochondrial proteins; or

(ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

5.  A method of calibrating a method of determining prognosis and/or treatment of choice of a patient suffering from AML, said method comprising

(d) determining the protein expression levels of a set of proteins in a cohort of patients suffering from AML;
(e) comparing expression levels of individual patients to average expression levels of said set of proteins in said cohort;
(f) correlating expression levels which deviate from said average expression levels with observed overall survival and/or response to chemotherapy;

wherein said set of proteins comprises or consists of

(iii) at least three mitochondrial proteins; or
(iv) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

6.  A method of determining prognosis and/or response to chemotherapy of a patient suffering from AML, said method comprising

(d) determining the protein expression levels of a set of proteins in said patient;
(e) comparing said expression levels to the average expression levels of said set of proteins in a cohort of AML patients, wherein said average expression levels may be retrieved in a database;
(f) assigning bad prognosis and/or poor response to chemotherapy to said patient if said expression levels deviate from said average expression levels, wherein said set of proteins comprises or consists of
(iii) at least three mitochondrial proteins, wherein said mitochondrial proteins are predominantly elevated as compared to said average expression levels; or
(iv) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels.

7.  A method of selecting an AML patient for a specific therapy, said method comprising

(d) determining the protein expression levels of a set of proteins in said patient;
(e) comparing said expression levels to the average expression levels of said set of proteins in a cohort of AML patients, wherein said average expression levels may be retrieved in a database;
(f) selecting said patient for said specific therapy if said expression levels deviate from said average expression levels;

wherein said set of proteins comprises or consists of

(iii) at least three mitochondrial proteins, wherein said mitochondrial proteins are predominantly elevated as compared to said average expression levels; or
(iv) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels;

and wherein said specific therapy comprises or consists of the administration to said patient of an inhibitor of mitochondrial function, preferably of the electron transport chain (ETC), more preferably of ETC complex I.

8. The method of claim 7, wherein said mitochondrial inhibitor targeting mitochondrial proteins, wherein is selected from Venetoclax, Tigecycline, Mubritinib, Rotenone, Metformin, Deguelin, IACS-010759, Oligomycin, Gboxin, Gamitrinib, TRO40303, cyclosporin A, Paclitaxel, mitocans, PBR-agonists including diazepam analogs, 3-bromopyruvate, Anthracyclines, arsenic trioxide, and 4-Quinolones such as ciprofloxacin.

9. The method of any one of claims 1 to 8, wherein said method is an in vitro method and/or said determining is performed with (a) sample(s) obtained from said cohort and/or said patient.

10. An agent for use in a method of treating a sub-form of AML, wherein said sub-form of AML is **characterized by** deviant expression of a set of proteins as compared to the average expression of said set of proteins in a cohort of AML patients, wherein said set of proteins comprises or consists of

    (i) at least three mitochondrial proteins, wherein said mitochondrial proteins are predominantly elevated as compared to said average expression levels; or
    (ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR, wherein expression levels of DLD, ETFB, NUP160, RDX, LDHA, FAM49B, GRB2, MAPRE1, DAD1, NUP210, GLRX3, APOOL, COQ7, IMMT, EEF2, LPCAT3, CSK, MRPL19, NUP88 and nucleoprotein TPR, to the extent they are determined, are elevated as compared to said average expression levels, and expression levels of COX6C, SIRT3, AGPAT3, DHRS1, AIFM1, FASN, and USO1, to the extent they are determined, are decreased as compared to said average expression levels;

    and wherein said agent is an inhibitor of mitochondrial function, preferably of the electron transport chain (ETC), more preferably of ETC complex I.

11. The agent for use of claim 10, wherein said method of treating does not include chemotherapy.

12. A method of identifying a candidate therapeutic target for AML, said method comprising

    (a) determining the expression level of at least one protein in a sample obtained from an AML patient;
    (b) comparing said expression level to the average expression level of said at least one protein in a cohort of AML patients; and
    (c) selecting a protein with a deviant, preferably an elevated expression level as determined in step (b) as candidate therapeutic target.

13. A method of identifying an agent suitable for AML therapy or as a lead compound for an AML therapeutic, said method comprising the method of claim 12, and bringing a candidate compound into contact with a selected target, wherein modified activity, preferably reduced activity of said target compared to activity of said target in absence of said compound is indicative of said compound being a suitable agent or lead compound.

14. The method of any one of the preceding claims, wherein said determining is effected by mass spectrometry (MS), Immuno-histochemistry, ELISA, Flow cytometry, antibodies, Western blotting, biosensors, microscopy techniques combined with benchtop fluidics systems such as Akoya's CODEX® instrument, and/or microfluidic immunoassay chips, preferably by MS.

15. A device configured for determining protein expression levels of a set of proteins, wherein said set of proteins comprises or consists of

    (i) at least three mitochondrial proteins; or
    (ii) at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

16. A kit comprising or consisting of at least three antibodies, wherein each of said antibodies is specific for one protein selected from a set of proteins, wherein said set of proteins consists of at least three proteins selected from DLD, ETFB, COX6C, NUP160, RDX, LDHA, FAM49B, GRB2, MAPRE1, SIRT3, DAD1, NUP210, GLRX3, APOOL, COQ7, IMMT, AGPAT3, DHRS1, EEF2, LPCAT3, AIFM1, FASN, USO1, CSK, MRPL19, NUP88 and nucleoprotein TPR.

17. A computer-implemented method of training a predictor of overall survival or response to chemotherapy of an AML patient and/or of effective treatment of said patient, said method comprising providing a training data set to a machine learning model, wherein said training data set associates protein expression levels as defined in claim 4 with overall survival, response to chemotherapy and/or effective treatment.

18. The method, the use, the agent for use, the device, the kit, or the computer-implemented method of any one of the preceding claims, wherein said at least three proteins are selected from CYRIB, CSK, MRPL19, NUP88 and nucleoprotein TPR.

Figure 1

# Figure 2

Figure 3

## Figure 4

# Figure 5

Figure 6

Europäisches
Patentamt

European
Patent Office

Office européen
des brevets

# EUROPEAN SEARCH REPORT

Application Number

EP 21 21 6747

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LUCZAK MAGDALENA ET AL: "Comparative proteomics in acute myeloid leukemia", WSPÓ&LSTROK;CZESNA ONKOLOGIA, vol. 2, 29 May 2012 (2012-05-29), pages 95-103, XP055926305, ISSN: 1428-2526, DOI: 10.5114/wo.2012.28787 Retrieved from the Internet: URL:https://www.ncbi.nlm.nih.gov/pmc/articles/PMC3687393/pdf/WO-16-18651.pdf> [retrieved on 2022-06-23] * The whole document; in particular the section "Proteomics in acute myeloid leukemia diagnostics". * | 1-3 | INV. G01N33/574 |
| X | HU CHENYUE W ET AL: "Standard Proteomic Analysis (SPA) in AML: An Integrated Procedure for Discovering Hypoxia and Angiogenesis Patterns", BLOOD, AMERICAN SOCIETY OF HEMATOLOGY, US, vol. 124, no. 21, 6 December 2014 (2014-12-06), pages 1056-1059, XP086739964, ISSN: 0006-4971, DOI: 10.1182/BLOOD.V124.21.1056.1056 [retrieved on 2021-06-25] * The whole document; in particular page 2 second paragraph, the section "results" and Figure 1A. * | 1-3 | |
| | -/-- | | TECHNICAL FIELDS SEARCHED (IPC) G01N |

The present search report has been drawn up for all claims

| Place of search | Date of completion of the search | Examiner |
|---|---|---|
| Munich | 28 June 2022 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number

EP 21 21 6747

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LÓPEZ-PEDRERA CHARY ET AL: "Proteomic analysis of acute myeloid leukemia: Identification of potential early biomarkers and therapeutic targets", PROTEOMICS, vol. 6, 18 April 2006 (2006-04-18), pages S293-S299, XP055926284, DOI: 10.1002/pmic.200500384 * The whole document; in particular the abstract. * | 1-3 | |
| X | AASEBØ ELISE ET AL: "Global Cell Proteome Profiling, Phospho-signaling and Quantitative Proteomics for Identification of New Biomarkers in Acute Myeloid Leukemia Patients", CURRENT PHARMACEUTICAL BIOTECHNOLOGY, vol. 17, no. 1, 1 January 2016 (2016-01-01), pages 52-70, XP055926308, DOI: 10.2174/1389201016666150826115626 Retrieved from the Internet: URL:https://www.ncbi.nlm.nih.gov/pmc/articles/PMC5388801/pdf/CPB-17-52.pdf> [retrieved on 2022-06-23] * The whole document; in particular the abstract and section "3. MS-based methodologies for the study the AML proteome" and section "5. proteomic contributions to unravel the AML proteome". * | 1-3 | TECHNICAL FIELDS SEARCHED (IPC) |

-/--

The present search report has been drawn up for all claims

| Place of search | Date of completion of the search | Examiner |
|---|---|---|
| Munich | 28 June 2022 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | POETA GIOVANNI DEL ET AL: "The Amount of Mitochondrial Apoptosis Exerts Prognostic Impact on Normal Karyotype Acute Myeloid Leukemia", BLOOD, AMERICAN SOCIETY OF HEMATOLOGY, US, vol. 112, no. 11, 16 November 2008 (2008-11-16), page 1481, XP086682530, ISSN: 0006-4971, DOI: 10.1182/BLOOD.V112.11.1481.1481 * The whole document; in particular page 1 second paragraph and first sentence of the third paragraph spanning to page 2. * ----- | 4-11,14, 16,17 | |
| X | Kern Beate: "Analysis of Helicobacter pylori VacA-containing vacuoles and VacA intracellular trafficking", Dissertation, 28 June 2015 (2015-06-28), pages 1-197, XP055933884, Retrieved from the Internet: URL:https://edoc.ub.uni-muenchen.de/18405/1/Kern_Beate.pdf [retrieved on 2022-06-21] * The whole document; in particular Table 6.2 * ----- | 15 | |
| X | ZIQING CHEN ET AL: "Current applications of antibody microarrays", CLINICAL PROTEOMICS, SPRINGER, US, vol. 15, no. 7, 28 February 2018 (2018-02-28), pages 1-15, XP021253958, ISSN: 1542-6416, DOI: 10.1186/S12014-018-9184-2 * The whole document. * ----- | 16 | |

TECHNICAL FIELDS SEARCHED (IPC)

The present search report has been drawn up for all claims

| Place of search | Date of completion of the search | Examiner |
|---|---|---|
| Munich | 28 June 2022 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3(completely); 4-11, 14-17(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

The Search Division considers that the present European patent application does not comply with the requirements of unity of invention and relates to several inventions or groups of inventions, namely:

```
1. claims: 1-3(completely); 4-11, 14-17(partially)

    Use of at least the proteins DLD, ETFB, and COX6C for the
    assessment of acute myeloid leukemia.
                        ---


2-50. claims: 4-11, 14-18(all partially)

    Use of any remaining combination of at least three proteins
    specified in claims 4-7, 10, 15, and 16 for the assessment
    of acute myeloid leukemia.
    Note: the number of inventions is just a rough estimation.
                        ---


51. claims: 12, 13(completely); 14(partially)

    Method of identifying a lead compound comprising determining
    the expression level of at least one protein in a sample
    obtained from an acute myeloid leukemia patient. Methods
    based thereon.
                        ---
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TYANOVA et al.** *Nature Methods,* 2016, vol. 13, 731-740
- **COX ; MANN.** *BMC Bioinformatics,* 2012, vol. 13 (16), S12
- **CHEN, TIANQI ; CARLOS GUESTRIN.** Xgboost: A scalable tree boosting system. *Proceedings of the 22nd acm sigkdd international conference on knowledge discovery and data mining,* 2016
- **KATZMAN, JARED L. et al.** DeepSurv: personalized treatment recommender system using a Cox proportional hazards deep neural network. *BMC medical research methodology,* 2018, vol. 18 (1), 1-12
- **LIN, DANYU Y. ; LEE-JEN WEI.** The robust inference for the Cox proportional hazards model. *Journal of the American statistical Association,* 1989, vol. 84 (408), 1074-1078
- **DE IORIO, MARIA et al.** Bayesian nonparametric nonproportional hazards survival modeling. *Biometrics,* 2009, vol. 65 (3), 762-771
- **ARGELAGUET, R. ; VELTEN, B. ; ARNOL, D. ; DIETRICH, S. ; ZENZ, T. ; MARIONI, J.C. ; BUETTNER, F. ; HUBER, W. ; STEGLE, O.** Multi-Omics Factor Analysis-a framework for unsupervised integration of multi-omics data sets. *Mol. Syst. Biol.,* 2018, vol. 14
- **ARMSTRONG, S.A. ; STAUNTON, J.E. ; SILVERMAN, L.B. ; PIETERS, R. ; DEN BOER, M.L. ; MINDEN, M.D. ; SALLAN, S.E. ; LANDER, E.S. ; GOLUB, T.R. ; KORSMEYER, S.J.** MLL translocations specify a distinct gene expression profile that distinguishes a unique leukemia. *Nat. Genet.,* 2002, vol. 30, 41-47
- **BERNT, K.M. ; ARMSTRONG, S.A.** Targeting epigenetic programs in MLL-rearranged leukemias. *Hematology Am. Soc. Hematol. Educ. Program,* 2011, vol. 2011, 354-360
- **BHATT, S. ; PIOSO, M.S. ; OLESINSKI, E.A. ; YILMA, B. ; RYAN, J.A. ; MASHAKA, T. ; LEUTZ, B. ; ADAMIA, S. ; ZHU, H. ; KUANG, Y. et al.** Reduced Mitochondrial Apoptotic Priming Drives Resistance to BH3 Mimetics in Acute Myeloid Leukemia. *Cancer Cell,* 2020
- **BLOMEN, V.A. ; MÁJEK, P. ; JAE, L.T. ; BIGENZAHN, J.W. ; NIEUWENHUIS, J. ; STARING, J. ; SACCO, R. ; VAN DIEMEN, F.R. ; OLK, N. ; STUKALOV, A. et al.** Gene essentiality and synthetic lethality in haploid human cells. *Science,* 2015, vol. 350 (80), 1092-1096

- **BOHNENBERGER, H. ; KADERALI, L. ; STROBEL, P. ; YEPES, D. ; PLESSMANN, U. ; DHARIA, N. V ; YAO, S. ; HEYDT, C. ; MERKELBACH-BRUSE, S. ; EMMERT, A. et al.** Comparative proteomics reveals a diagnostic signature for pulmonary head-and-neck cancer metastasis. *EMBO Mol. Med.,* 2018, vol. 10
- **CAPES-DAVIS, A. ; REID, Y.A. ; KLINE, M.C. ; STORTS, D.R. ; STRAUSS, E. ; DIRKS, W.G. ; DREXLER, H.G. ; MACLEOD, R.A.F. ; SYKES, G. ; KOHARA, A. et al.** Match criteria for human cell line authentication: Where do we draw the line?. *Int. J. Cancer,* 2013, vol. 132, 2510-2519
- **CHAO, M.P. ; TAKIMOTO, C.H. ; FENG, D.D. ; MCKENNA, K. ; GIP, P. ; LIU, J. ; VOLKMER, J.P. ; WEISSMAN, I.L. ; MAJETI, R.** Therapeutic Targeting of the Macrophage Immune Checkpoint CD47 in Myeloid Malignancies. *Front. Oncol.,* 2020, vol. 9
- **CHOPRA, S. ; RAMKISSOON, K. ; ANDERSON, D.C.** A systematic quantitative proteomic examination of multidrug resistance in Acinetobacter baumannii. *J. Proteomics,* 2013, vol. 84, 17-39
- **COSCIA, F. ; LENGYEL, E. ; DURAISWAMY, J. ; ASHCROFT, B. ; BASSANI-STERNBERG, M. ; WIERER, M. ; JOHNSON, A. ; WROBLEWSKI, K. ; MONTAG, A. ; YAMADA, S.D. et al.** Multi-level Proteomics Identifies CT45 as a Chemosensitivity Mediator and Immunotherapy Target in Ovarian Cancer. *Cell,* 2018, vol. 175, 159-170.e16
- **COX, J. ; HEIN, M.Y. ; LUBER, C.A. ; PARON, I. ; NAGARAJ, N. ; MANN, M.** Accurate proteomewide label-free quantification by delayed normalization and maximal peptide ratio extraction, termed MaxLFQ. *Mol. Cell. Proteomics,* 2014, vol. 13, 2513-2526
- **DEMPSTER, J.M. ; ROSSEN, J. ; KAZACHKOVA, M. ; PAN, J. ; KUGENER, G. ; ROOT, D.E. ; TSHERNIAK, A.** Extracting Biological Insights from the Project Achilles Genome-Scale CRISPR Screens in Cancer Cell Lines. *BioRxiv 720243,* 2019
- **DINARDO, C.D. ; STEIN, E.M. ; DE BOTTON, S. ; ROBOZ, G.J. ; ALTMAN, J.K. ; MIMS, A.S. ; SWORDS, R. ; COLLINS, R.H. ; MANNIS, G.N. ; POLLYEA, D.A. et al.** Durable Remissions with Ivosidenib in IDH1-Mutated Relapsed or Refractory AML. *N. Engl. J. Med. NEJM,* 2018, 1716984

- **DINARDO, C.D. ; JONAS, B.A. ; PULLARKAT, V. ; THIRMAN, M.J. ; GARCIA, J.S. ; WEI, A.H. ; KONOPLEVA, M. ; DÖHNER, H. ; LETAI, A. ; FENAUX, P. et al.** Azacitidine and Venetoclax in Previously Untreated Acute Myeloid Leukemia. *N. Engl. J. Med.,* 2020, vol. 383, 617-629
- **DOENCH, J.G. ; FUSI, N. ; SULLENDER, M. ; HEGDE, M. ; VAIMBERG, E.W. ; DONOVAN, K.F. ; SMITH, I. ; TOTHOVA, Z. ; WILEN, C. ; ORCHARD, R. et al.** Optimized sgRNA design to maximize activity and minimize off-target effects of CRISPR-Cas9. *Nat. Biotechnol.,* 2016, vol. 34, 184-191
- **DÖHNER, H. ; ESTEY, E. ; GRIMWADE, D. ; AMADORI, S. ; APPELBAUM, F.R. ; BÜCHNER, T. ; DOMBRET, H. ; EBERT, B.L. ; FENAUX, P. ; LARSON, R.A. et al.** Diagnosis and management of AML in adults: 2017 ELN recommendations from an international expert panel. *Blood,* 2017, vol. 129, 424-447
- **FARGE, T. ; SALAND, E. ; DE TONI, F. ; AROUA, N. ; HOSSEINI, M. ; PERRY, R. ; BOSC, C. ; SUGITA, M. ; STUANI, L. ; FRAISSE, M. et al.** Chemotherapy-Resistant Human Acute Myeloid Leukemia Cells Are Not Enriched for Leukemic Stem Cells but Require Oxidative Metabolism. *Cancer Discov,* 2017, vol. 7, 716-735
- **FROST, H.R. ; LI, Z. ; MOORE, J.H.** Principal component gene set enrichment (PCGSE). *BioData Min,* 2015, vol. 8, 25
- **GEIGER, T. ; COX, J. ; OSTASIEWICZ, P. ; WISNIEWSKI, J.R. ; MANN, M.** Super-SILAC mix for quantitative proteomics of human tumor tissue. *Nat. Methods,* 2010, vol. 7, 383-385
- **GUO, X. ; ZHONG, W. ; CHEN, Y. ; ZHANG, W. ; REN, J. ; GAO, A.** Benzene metabolites trigger pyroptosis and contribute to haematotoxicity via TET2 directly regulating the Aim2/Casp1 pathway. *EBioMedicine,* 2019, vol. 47, 578-589
- **HART, T. ; BROWN, K.R. ; SIRCOULOMB, F. ; ROTTAPEL, R. ; MOFFAT, J.** Measuring error rates in genomic perturbation screens: gold standards for human functional genomics. *Mol. Syst. Biol.,* 2014, vol. 10, 733
- **HART, T. ; CHANDRASHEKHAR, M. ; AREGGER, M. ; STEINHART, Z. ; BROWN, K.R. ; MACLEOD, G. ; MIS, M. ; ZIMMERMANN, M. ; FRADET-TURCOTTE, A. ; SUN, S. et al.** High-Resolution CRISPR Screens Reveal Fitness Genes and Genotype-Specific Cancer Liabilities. *Cell,* 2015, vol. 163, 1515-1526
- **HARTMANN, S. ; AGOSTINELLI, C. ; KLAPPER, W. ; KORKOLOPOULOU, P. ; KOCH, K. ; MARAFIOTI, T. ; PICCALUGA, P.P. ; PATSOURIS, E. ; PILERI, S. ; HANSMANN, M.L.** Revising the historical collection of epithelioid cell-rich lymphomas of the Kiel Lymph Node Registry: What is Lennert's lymphoma nowadays?. *Histopathology,* 2011, vol. 59, 1173-1182
- **HUANG, Y. ; SITWALA, K. ; BRONSTEIN, J. ; SANDERS, D. ; DANDEKAR, M. ; COLLINS, C. ; ROBERTSON, G. ; MACDONALD, J. ; CEZARD, T. ; BILENKY, M. et al.** Identification and characterization of Hoxa9 binding sites in hematopoietic cells. *Blood,* 2012, vol. 119, 388-398
- **HUBER, W. ; CAREY, V.J. ; GENTLEMAN, R. ; ANDERS, S. ; CARLSON, M. ; CARVALHO, B.S. ; BRAVO, H.C. ; DAVIS, S. ; GATTO, L. ; GIRKE, T. et al.** Orchestrating high-throughput genomic analysis with Bioconductor. *Nat. Methods,* 2015, vol. 12, 115-121
- **JAYAVELU, A.K. ; SCHNÖDER, T.M. ; PERNER, F. ; HERZOG, C. ; MEILER, A. ; KRISHNAMOORTHY, G. ; HUBER, N. ; MOHR, J. ; EDELMANN-STEPHAN, B. ; AUSTIN, R. et al.** Splicing factor YBX1 mediates persistence of JAK2-mutated neoplasms. *Nature,* 2020, vol. 588, 157-163
- **KAMGA, P.T. ; COLLO, G.D. ; RESCI, F. ; BAZZONI, R. ; MERCURI, A. ; QUAGLIA, F.M. ; TANASI, I. ; DELFINO, P. ; VISCO, C. ; BONIFACIO, M. et al.** Notch Signaling Molecules as Prognostic Biomarkers for Acute Myeloid Leukemia. *Cancers,* 2019, vol. 11, 1958
- **KO, M. ; AN, J. ; BANDUKWALA, H.S. ; CHAVEZ, L. ; ÄIJÖ, T. ; PASTOR, W.A. ; SEGAL, M.F. ; LI, H. ; KOH, K.P. ; LÄHDESMÄKI, H. et al.** Modulation of TET2 expression and 5-methylcytosine oxidation by the CXXC domain protein IDAX. *Nature,* 2013, vol. 497, 122-126
- **KULAK, N.A. ; PICHLER, G. ; PARON, I. ; NAGARAJ, N. ; MANN, M.** Minimal, encapsulated proteomic-sample processing applied to copy-number estimation in eukaryotic cells. *Nat. Methods,* 2014, vol. 11, 319-324
- **KUŽELOVÁ, K. ; BRODSKÁ, B. ; SCHETELIG, J. ; ROLLIG, C. ; RÁČIL, Z. ; WALZ, J.S. ; HELBIG, G. ; FUCHS, O. ; VRANÁ, M. ; PECHERKOVÁ, P. et al.** Association of HLA class I type with prevalence and outcome of patients with acute myeloid leukemia and mutated nucleophosmin. *PLoS One,* 2018, vol. 13
- **LACHOWIEZ, C.A. ; LOGHAVI, S. ; KADIA, T.M. ; DAVER, N. ; BORTHAKUR, G. ; PEMMARAJU, N. ; NAQVI, K. ; ALVARADO, Y. ; YILMAZ, M. ; SHORT, N. et al.** Outcomes of older patients with NPM1-mutated AML: Current treatments and the promise of venetoclax-based regimens. *Blood Adv,* 2020, vol. 4, 1311-1320
- **LAMBERT, S.A. ; JOLMA, A. ; CAMPITELLI, L.F. ; DAS, P.K. ; YIN, Y. ; ALBU, M. ; CHEN, X. ; TAIPALE, J. ; HUGHES, T.R. ; WEIRAUCH, M.T.** The Human Transcription Factors. *Cell,* 2018, vol. 172, 650-665

- **LIBERZON, A. ; SUBRAMANIAN, A. ; PINCH-BACK, R. ; THORVALDSDÓTTIR, H. ; TAMAYO, P. ; MESIROV, J.P.** Molecular signatures database (MSigDB) 3.0. *Bioinformatics,* 2011, vol. 27, 1739-1740
- **LIBERZON, A. ; BIRGER, C. ; THORVALDSDÓTTIR, H. ; GHANDI, M. ; MESIROV, J.P. ; TAMAYO, P.** The Molecular Signatures Database Hallmark Gene Set Collection. *Cell Syst,* 2015, vol. 1, 417-425
- **LOVE, M.I. ; HUBER, W. ; ANDERS, S.** Moderated estimation of fold change and dispersion for RNA-seq data with DESeq2. *Genome Biol,* 2014, vol. 15
- **MANNING, G. ; WHYTE, D.B. ; MARTINEZ, R. ; HUNTER, T. ; SUDARSANAM, S.** The protein kinase complement of the human genome. *Science,* 2002, vol. 298 (80), 1912-1934
- **MCLNNES, L. ; HEALY, J. ; MELVILLE, J.** *UMAP: Uniform Manifold Approximation and Projection for Dimension Reduction,* 2018
- **MEIER, J.A. ; LARNER, A.C.** Toward a new STATe: The role of STATs in mitochondrial function. *Semin. Immunol.,* 2014, vol. 26, 20-28
- **MEYERS, R.M. ; BRYAN, J.G. ; MCFARLAND, J.M. ; WEIR, B.A. ; SIZEMORE, A.E. ; XU, H. ; DHARIA, N. V ; MONTGOMERY, P.G. ; COWLEY, G.S. ; PANTEL, S. et al.** Computational correction of copy number effect improves specificity of CRISPR - Cas9 essentiality screens in cancer cells. *Nat. Publ. Gr.,* 2017
- **PABST, T. ; MUELLER, B.U.** Complexity of CEBPA dysregulation in human acute myeloid leukemia. *Clin. Cancer Res.,* 2009, vol. 15, 5303-5307
- **PACZULLA, A.M. ; ROTHFELDER, K. ; RAFFEL, S. ; KONANTZ, M. ; STEINBACHER, J. ; WANG, H. ; TANDLER, C. ; MBARGA, M. ; SCHAEFER, T. ; FALCONE, M. et al.** Absence of NKG2D ligands defines leukaemia stem cells and mediates their immune evasion. *Nature,* 2019, vol. 572, 254-259
- **PAPAEMMANUIL, E. ; GERSTUNG, M. ; BULL-INGER, L. ; GAIDZIK, V.I. ; PASCHKA, P. ; ROBERTS, N.D. ; POTTER, N.E. ; HEUSER, M. ; THOL, F. ; BOLLI, N. et al.** Genomic Classification and Prognosis in Acute Myeloid Leukemia. *N. Engl. J. Med.,* 2016, vol. 374, 2209-2221
- **PEI, S. ; MINHAJUDDIN, M. ; ADANE, B. ; KHAN, N. ; STEVENS, B.M. ; MACK, S.C. ; LAI, S. ; RICH, J.N. ; INGUVA, A. ; SHANNON, K.M. et al.** AMPK/FIS1-Mediated Mitophagy Is Required for Self-Renewal of Human AML Stem Cells. *Cell Stem Cell,* 2018, vol. 23, 86-100.e6
- **PHIPSON, B. ; LEE, S. ; MAJEWSKI, I.J. ; ALEXANDER, W.S. ; SMYTH, G.K.** Robust hyperparameter estimation protects against hypervariable genes and improves power to detect differential expression. *Ann. Appl. Stat.,* 2016, vol. 10, 946-963
- **PICELLI, S. ; FARIDANI, O.R. ; BJÖRKLUND, Å.K. ; WINBERG, G. ; SAGASSER, S. ; SANDBERG, R.** Full-length RNA-seq from single cells using Smart-seq2. *Nat. Protoc.,* 2013, vol. 91 (9), 171-181
- **PJ, V. ; RG, V. ; MA, B. ; CA, E. ; S, B. VAN W. VAN D.-K. ; JM, B. ; HB, B. ; MJ, M. ; PJ, VAN DER S. ; B, L. et al.** Prognostically useful gene-expression profiles in acute myeloid leukemia. *N. Engl. J. Med.,* 2004, vol. 350, 1617-1628
- **PTASINSKA, A. ; PICKIN, A. ; ASSI, S.A. ; CHIN, P.S. ; AMES, L. ; AVELLINO, R. ; GROSCHEL, S. ; DELWEL, R. ; COCKERILL, P.N. ; OSBORNE, C.S. et al.** RUNX1-ETO Depletion in t(8;21) AML Leads to C/EBPα-and AP-1-Mediated Alterations in Enhancer-Promoter Interaction. *Cell Rep,* 2019, vol. 28, 3022-3031.e7
- *R: A Language and Environment for Statistical Computing,* 2020
- **RATH, S. ; SHARMA, R. ; GUPTA, R. ; AST, T. ; CHAN, C. ; DURHAM, T.J. ; GOODMAN, R.P. ; GRABAREK, Z. ; HAAS, M.E. ; HUNG, W.H.W. et al.** MitoCarta3.0: An updated mitochondrial proteome now with sub-organelle localization and pathway annotations. *Nucleic Acids Res,* 2021, vol. 49, D1541-D1547
- **RITZ, C. ; BATY, F. ; STREIBIG, J.C. ; GERHARD, D.** Dose-Response Analysis Using R. *PLoS One,* 2015, vol. 10, e0146021
- **SANJANA, N.E. ; SHALEM, O. ; ZHANG, F.** Improved vectors and genome-wide libraries for CRISPR screening. *Nat. Methods,* 2014, vol. 11, 783-784
- **SHAFER, D. ; GRANT, S.** Update on rational targeted therapy in AML. *Blood Rev,* 2016, vol. 30, 275-283
- **SOLH, M. ; YOHE, S. ; WEISDORF, D. ; USTUN, C.** Core-binding factor acute myeloid leukemia: Heterogeneity, monitoring, and therapy. *Am. J. Hematol.,* 2014, vol. 89, 1121-1131
- **STONE, R.M. ; MANDREKAR, S.J. ; SANFORD, B.L. ; LAUMANN, K. ; GEYER, S. ; BLOOMFIELD, C.D. ; THIEDE, C. ; PRIOR, T.W. ; DÖHNER, K. ; MARCUCCI, G. et al.** Midostaurin plus Chemotherapy for Acute Myeloid Leukemia with a FLT3 Mutation. *N. Engl. J. Med.,* 2017, vol. 377, 454-464
- **SUBRAMANIAN, A. ; TAMAYO, P. ; MOOTHA, V.K. ; MUKHERJEE, S. ; EBERT, B.L. ; GILLETTE, M.A. ; PAULOVICH, A. ; POMEROY, S.L. ; GOLUB, T.R. ; LANDER, E.S. et al.** Gene set enrichment analysis: A knowledge-based approach for interpreting genome-wide expression profiles. *Proc. Natl. Acad. Sci. U. S. A.,* 2005, vol. 102, 15545-15550
- **TERRY M. THERNEAU ; PATRICIA M. GRAMBSCH.** Modeling Survival Data: Extending the {C}ox Model. Springer, 2000

- **THIEDE, C. ; KOCH, S. ; CREUTZIG, E. ; STEUDEL, C. ; ILLMER, T. ; SCHAICH, M. ; EHNINGER, G.** Prevalence and prognostic impact of NPM1 mutations in 1485 adult patients with acute myeloid leukemia (AML). *Blood,* 2006, vol. 107, 4011-4020
- **TYANOVA, S. ; TEMU, T. ; COX, J.** The MaxQuant computational platform for mass spectrometry-based shotgun proteomics. *Nat. Protoc.,* 2016, vol. 11, 2301-2319
- **TYANOVA, S. ; TEMU, T. ; SINITCYN, P. ; CARLSON, A. ; HEIN, M.Y. ; GEIGER, T. ; MANN, M. ; COX, J.** The Perseus computational platform for comprehensive analysis of (prote)omics data. *Nat. Methods,* 2016, vol. 13, 731-740
- **DAVID S.** *THE FIRST-IN-CLASS ANTI-CD47 ANTIBODY MAGROLIMAB COMBINED WITH....* EHA Library, 12 June 2020, 295007